# EUROPÄISCHE PATENTSCHRIFT

(11) **EP 0 368 167 B1**
(45) Veröffentlichungstag und Bekanntmachung des Hinweises auf die Patenterteilung: **08.02.1995**
(21) Anmeldenummer: 89120390.3
(22) Anmeldetag: 03.11.1989
(51) Int. Cl.: C12N 15/29, C12N 15/82, A01H 5/00

(54) **Verwundungsstimulierte DNA-Sequenz aus Solanum tuberosum und ihre Verwendung**
Injury-stimulated DNA sequence from Solanum tuberosum, and its use
Séquence d'ADDN de solanum tuberosum induite par blessure et son utilisation

(30) Priorität: 07.11.1988 DE 3837752
(43) Veröffentlichungstag der Anmeldung: 16.05.1990
(73) Patentinhaber: Max-Planck-Gesellschaft zur Förderung der Wissenschaften e.V., D-37073 Göttingen (DE)
(72) Erfinder: Logemann, Jürgen, D-5042 Erftstadt (DE); Willmitzer, Lothar, D-1000 Berlin 39 (DE); Schell, Josef, D-5000 Köln 30 (DE)
(74) Vertreter: von Hellfeld, Axel, Dr. Dipl.-Phys.,

(56) Entgegenhaltungen:
- PROC. NATL. ACAD. SCI. USA, Band 85, Februar 1988, Seiten 1136-1140; J. LOGEMANN et al.: "Differential expression of genes in potato tubers after wounding"
- PROC. NATL. ACAD. SCI. USA, Band 84, Februar 1987, Seiten 744-748; R.W. THORNBURG et al.: "Wound-inducible expression of a potato inhibitor II-chloramphenicol acetyltransferase gene fusion in transgenic tobacco plants"
- THE EMBO JOURNAL, Band 6, Nr. 2, Februar 1987, Seiten 303-306, IRL Press Ltd, Eynsham Oxford, GB; J.J. SANCHEZ-SERRANO, et al.: "Wound-induced expression of a potato proteinase inhibitor II gene in transgenic tobacco plants"
- PLANT MOLECULAR BIOLOGY, Band 8, 1987, Seiten 199-207; T.E. CLEVELAND et al.: "Molecular characterization of a wound-inducible inhibitor I gene from potato and the processing of its mRNA and protein"
- SCIENCE, Band 237, 4. September 1987, Seiten 1176-1183; J. St. SCHELL: "Transgenic plants as tools to study the molecular organization of plant genes"
- CHEMICAL ABSTRACTS, Band 101, 1984, Seite 202, Zusammenfassung Nr. 223982b, Columbus, Ohio, US; A.D. SHIRRAS et al.: "Molecular cloning and characterization of cDNAs complementary to mRNAs from wounded potato (Solanum tuberosum) tuber tissue"

## Beschreibung

Die Erfindung betrifft verwundungsstimulierte DNA aus Solanum tuberosum sowie Teile davon, deren Verwendung zur Expression von Genprodukten in höheren Pflanzen bei Verwundung oder pathogenem Befall, diese enthaltende DNA-Übertragungsvektoren und diese enthaltende Pflanzen oder Pflanzenteile.

Es ist bekannt, daß die mechanische Verwundung von Pflanzengewebe morphologische und physiologische Veränderungen bewirken kann. So steigt die Aktivität verschiedener Enzyme nach der Verwundung an, beispielsweise von Phenylalaninammoniumlyase und Peroxidasen in Kartoffelknollen, Extensin im Speichergewebe der Karotte, Fettsäuresynthetase in Kartoffelknollen und Proteinaseinhibitoren in Tomaten- und Kartoffelblättern. Bei einigen dieser Enzymen ist die Aktivitätszunahme mit erhöhten mRNA-Spiegeln verbunden. Eines der am besten verstandenen verwundungsinduzierten Gene ist der Proteinaseinhibitor in Tomaten und Kartoffeln; in verletzten Blättern ist die mRNA für dieses Gen deutlich erhöht.

Die verwundungsinduzierte Expression von Genen in Pflanzen ist von grundsätzlichem Interesse, da die hierbei wirksam werdenden Promotoren in der Lage sind, das zugehörige Strukturgen nach einer Verwundung spezifisch zu aktivieren. Derartige Promotoren, in Verbindung mit anderen Genen, die beispielsweise Resistenzen verleihen, antibiotisch wirksame Substanzen codieren oder die Heilung von Verletzungen fördern, sind natürlich von großem wirtschaftlichem Interesse, wenn sie in das genetische Material von anderen Pflanzen eingeschleust werden können.

Es wurde nun gefunden, daß es in verwundeten und/oder von Keimen befallenen Kartoffelpflanzen spezifisch zur Bildung von relativ hohen Konzentration von induzierter mRNA kommt. Diese mRNA konnte isoliert und charakterisiert werden; sie tritt in gesunden, unverwundeten Pflanzen nicht auf.

Gegenstand der Erfindung ist eine DNA-Sequenz aus Solanum tuberosum, die durch Verletzung und/oder Infektion mit pathogenen Keimen stimuliert werden, sowie deren Promotor- und Strukturgenteil. Gegenstand der Erfindung ist ferner die Verwendung dieser DNA-Sequenz zur Expression von Genprodukten in höheren Pflanzen bei Verletzung und/oder pathogenem Befall.

Gegenstand der Erfindung sind weiterhin DNA-Übertragungsvektoren mit darin insertierten DNA-Sequenzen, wie sie vorstehend definiert wurden. Schließlich sind Gegenstand der Erfindung Pflanzen- oder Pflanzenmaterialien, welche eine solche DNA-Sequenz enthalten.

Unter dem Begriff "transkriptionell aktiver Bereich" eines Gens, seines Promotor- und Strukturgenteils werden diejenigen Nukleotid-Sequenzen verstanden, die im Promotorteil des Gens für die Aktivierung des Strukturgenteils und im Strukturgenteil zur Expression eines wirksamen bzw. aktiven Genprodukts unbedingt benötigt werden.

Unter "Strukturgen" wird nicht nur das im homologen System der hier beschriebenen DNA-Sequenz aus Solanum tuberosum verstanden, sondern auch Strukturgene anderer Herkunft, also aus heterologen Systemen. Als Beispiele wird auf die CAT-, NPT- und GUS-Strukturgene hingewiesen, die mit dem hier beschriebenen verwendungsstimulierbaren Promotorteil fusioniert werden können und zur Expression des jeweiligen Strukturgens in der Kartoffel oder fremden Pflanzen verwandt werden können.

Als Übertragungsvektoren können jegliche geeignete DNA-Moleküle verwandt werden, die in das letztendlich aufnehmende Bakterium eingeführt werden können. Gewöhnlich sind dies Plasmide, wie sie im Materialienteil näher beschrieben sind. Die Insertionstechniken sind dem Fachmann bekannt.

Als Bakterien zur Aufnahme und Weitergabe des Übertragungsvektors können Bakterien der Gattung Agrobacterium, insbesondere Agrobacterium tumefaciens und Agrobakterium rhizogenes verwandt werden.

Die EP-A 122 791 beschreibt einen DNA-Übertragungsvektor, der T-DNA mit einem darin insertierten Pflanzengen umfaßt. Dieses Pflanzengen besteht aus einem Pflanzenpromotor und einem Pflanzenstrukturgen, wobei der Pflanzenpromotor an das 5′-Ende des Pflanzenstrukturgens angrenzt und das Pflanzenstrukturgen in der Transkriptionsrichtung hinter dem Pflanzenpromotor angeordnet ist. Die EP-A 122 791 beschreibt ausführlich Verfahren und Maßnahmen zur Insertierung von genetischem Material in DNA-Übertragungsvektoren, auf die hier ausdrücklich Bezug genommen wird.

Untersuchungen haben gezeigt, daß Erwinia-Bakterien latent auf Kartoffelpflanzen (Solanum tuberosum) vorhanden sind, und erst parallel zu einer Verwundung des Gewebes vorwiegend Stamm und Knolle der Kartoffel befallen. Dies wurde an Kartoffelknollen der Sorte Datura induziert, indem die Knolle in Scheiben geschnitten und gleichzeitig mit Erwinia carotovora ssp. atroseptica inkubiert wurde. Wie erwartet wiesen diese Knollen nach 18-stündiger Inkubation eine stark mazerierte Oberfläche auf. Die Isolation von mRNA aus dem darunter liegenden Gewebe war mit herkömmlichen Methoden nicht möglich. Hohe Kontamination der RNA durch Polysaccharide, schlechtes Präzipitations- und Lösungsverhalten der RNA, ein hoher Grad an RNA Degradation, geringe RNA-Ausbeute und hoher Arbeits- und Materialaufwand waren die Ursachen. Durch Variation und Neukombination bestehender Methoden konnte aber eine Technik entwickelt werden, die hohe Mengen an undegradierter RNA bei geringem Material-und Zeitaufwand auch aus polysaccharidhaltigem Gewebe zu isolieren imstande war (Logemann et al., Anal. Bioch. 163, 16, 1987).

Auf Basis gereinigter polyA⁺RNA aus verwundeten und zusätzlich mit Erwinia inkubierten Knollen der Kartoffelsorte Granola wurde eine cDNA-Bank aufgebaut. Durch differentielle Koloniehybridisierung von 4000 cDNA-Klonen gegen radioaktiv markierte RNA aus unverwundeten und Erwinia-verwundeten Knollen konnten 2 Klone, hier wun1 und wun2 genannt, identifiziert werden, deren komplementäre mRNA in Knollen verschiedener tetraploider Kartoffelsorten sowie der haploiden Sorte AM 80/5793 durch Verwundung induziert werden. Wun1-mRNA akkumuliert bereits 30 Minuten nach der mechanischen Verwundung einer Knolle und ist damit in verwundungsinduzierte Primärprozesse involviert, während wun2 erst 3,5 Stunden später induziert wird. Beide Klone zeigen auch 24 Stunden nach der Verwundung noch hohe Expressionswerte an. Wird zusätzlich zur mechanischen Verwundung die Knolle mit Erwinia carotovora ssp. atroseptica inkubiert, so finden keine Veränderungen am Expressionsschema statt. Insgesamt haben die Expressionsstudien an der Knolle ergeben, daß wun1- und wun2-mRNA's Gene repräsentieren, die nicht Erwinia-spezifisch induziert werden, sondern durch alle Vorgänge, die Zerstörung des Knollengewebes zur Folge haben, stimulierbar sind.

Wun1-mRNA akkumuliert in hohen Mengen in verwundeten Kartoffelknollen (mengenmäßig vergleichbar mit Proteinase-InhibitorII-mRNA in unverwundeten Kartoffelknollen, vgl. Sanchez-Serrano et a., Mol. Gen. Genet. 203, 15 (1986)), dagegen ist sie in unverwundeten Knollen nicht nachweisbar. Sie erscheint bereits 30 Minuten nach Verwundung mit einem Maximum ab der 4ten bis 24ten Stunde nach Verwundung. In geringeren Mengen war sie auch noch nach 48 Stunden nachweisbar.

Die Akkumulation von wun1- und wun2-mRNA nach Verwundung ist nicht auf die Knolle beschränkt, sondern findet auch in Blätter, Stämmen und Wurzel verschiedener tetraploider Kartoffelpflanzen mit ähnlicher Kinetik und Intensität statt.

Wun1-mRNA wird im Gegensatz zur wun2-mRNA auch ohne Verwundung in Blättern induziert, wenn diese mit kompatiblen Phytophtora infestans-Sporen besprüht werden. Dieses Ergebnis zeigt, daß wun1 nicht nur durch mechanische Verwendung induzierbar ist, sondern auch durch die Anwesenheit von (pilzlichen) Pathogenen.

Es ergaben sich Unterschiede in der Expression des wun1-Gens, je nachdem ob die Knollen unter aeroben Bedingungen verwundet wurden (Knollenscheiben wurden mit P-Puffer benetzt und für 18 Stunden inkubiert, sie waren somit weiterhin dem Luftsauerstoff ausgesetzt) oder unter anaeroben Bedingungen verwundet wurden (Knollenscheiben wurden in P-Puffer eingetaucht und in diesem luftsauerstoffarmen Zustand für 18 Stunden inkubiert). Wun1-mRNA wurde in aerob verwundeten Knollen höher exprimiert, als im entsprechenden anaeroben Ansatz.

Wichtig für die erfindungsgemäße Verwendung der Promotoren zur Expression von Genen ist, ob die nach Verwendung nachweisbare wun1- bzw. wun2-mRNA durch Neusynthese entsteht (transkriptionelle Regulation), oder aber constitutiv vorhanden ist, und nur im Falle einer Verwendung stabil vorliegt (posttranskriptionelle Regulation).

"Run-Off" Transkriptionstests konnten sowohl bei wun1 als auch bei wun2 transskriptionelle Regulation feststellen, was bedeutet, daß die Promotoren dieser Gene im Falle einer Verwendung für die Neusynthese verantwortlich sind.

Nach bekannten Verfahren wurden aus der tetraploiden Kartoffel "Granola" eine Reihe von Wun1-homologen cDNA-Klonen erhalten, von denen einige sequenziert wurden. Durch die Klonierung von wun1-25A2 in M13mp19-Phagen in beiden Orientierungen und partielle ExonukleaseIII-Verdauung konnte die Nukleotidsequenz bidirektional nach der Dideoxymethode bestimmt werden.

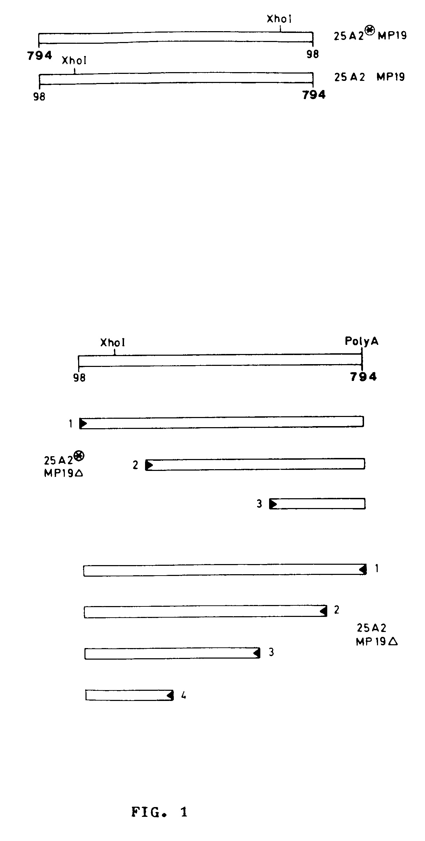

Fig. 1 zeigt die relative Größe der zur Sequenzierung verwandten Klone. Der Klon wun1-25A2 liegt in zwei Orientierungen in der PstI-Schnittstelle vor, die aufgrund der Lage des asymmetrischen XhoI Schnittstelle als 25A2*MP19 und 25A2MP19 bezeichnet werden. Durch sukzessive ExonukleaseIII-Verdauungen konnten unterschiedlich große Deletionsklone erhalten werden, die als Klone 25A2*MP19delta 1-3 bzw. 25A2MP19delta 1-4 zur Sequenzierung zur Verfügung standen.

Fig. 2 zeigt die Nukleotidsequenz des cDNA-Klons wun1-25A2. Alternative offene Lesearten sind unterstrichen.

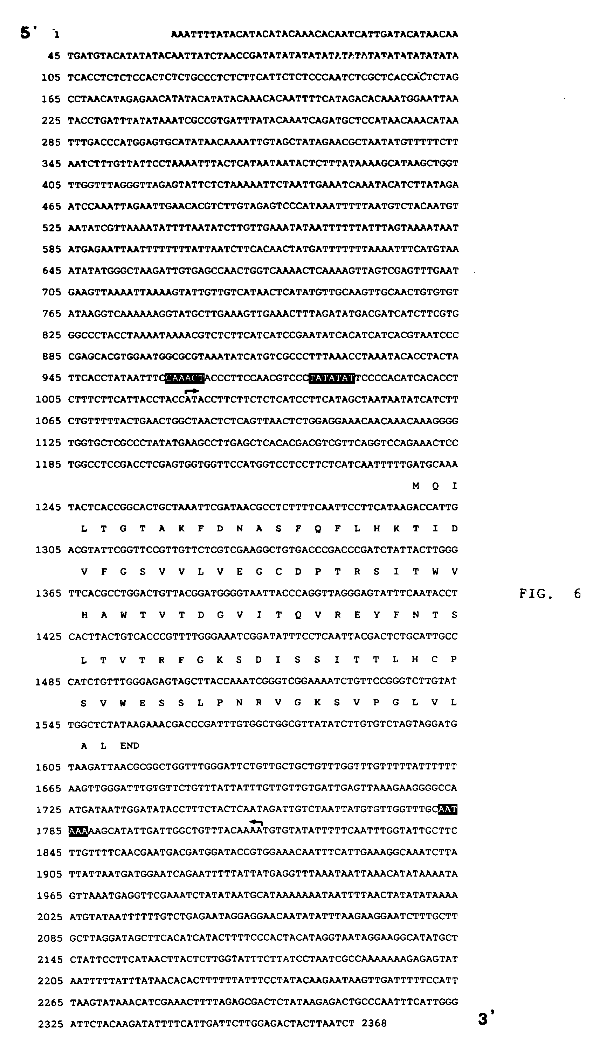

Die Größe des cDNA-Klons wun1-25A2 beträgt 711 bp. Am 3-Ende ist ein Poly-A-Schwanz von 14 bp zu finden (Position 697-711). Das größte offene Leseraster erstreckt sich über 105 Aminosäuren (Position 121-438). Weiter 5′-gelegen befinden sich außerdem zwei kurze offene Leseraster, die an Position 23 für 3 Aminosäuren kodieren und an Position 95 für 8 Aminosäuren kodieren (unterstrichen). Die beiden zuletzt angeführten offenen Leseraster unterscheiden sich jedoch vom Leseraster, das die 105 Aminosäuren kodiert. Unter Berücksichtigung der genomischen Sequenzdaten (Fig. 6) ist das für 105 Aminosäuren kodierende Leseraster als das des wun1-Proteins anzusehen. Der Translationsstartpunkt des wun1-Proteins TTTTTGATGCAA stimmt nur in geringem Maße mit der von Joshi, Nuc. Acids Res. 15, 6643, (1987) ermittelten Konsensussequenz für pflanzliche Translationsstarts TAAACAATGGCT überein.

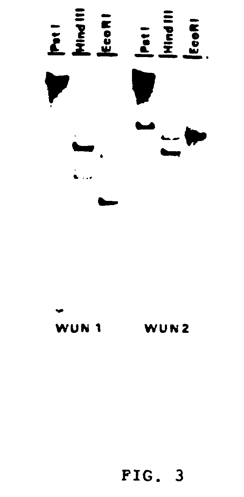

Die Untersuchung der genomischen Organisation des für wun1-mRNA kodierenden Gens in der haploiden Kartoffellinie AM80/5793 zeigt, daß dieses Gen in nur geringer Kopienzahl vorliegt. Die Hybridisierung radioaktiv markierter wun1-cDNA gegen HindIII-, PstI- und EcoRI-geschnittene Blatt-DNA führte in der "Southern-Blott"- Analyse zur Ausprägung von jeweils einer starken und 1 bis 2 schwachen Banden. Das Ergebnis der "Southern-Blot"-Analyse von wun1- und von wun2-cDNA ist in Fig. 3 dargestellt.

Die "Southern-Blot"-Analyse konnte in der haploiden Kartoffellinie AM80/5793 nur wenige für die Expression von wun1- und wun2-mRNA verantwortliche Gene nachweisen. Die Chance, ein aktives Gen und kein inaktives Pseudogen zu erhalten, war hier besonders hoch.

Die Präsenz nur einer ausgeprägten Bande im "Southern-Blot" der haploiden Kartoffellinie läßt auf eine geringe Anzahle an Genen pro Genom schließen. Betrachtet man dies im Zusammenhang mit einer verwundungssprezifischen und hohen Expression an wun1-mRNA in der haploiden Linie und der transkriptionellen Expressionsregulation, so sind hochaktive Gene wahrscheinlich.

Zur Anlage einer genomischen Bank wurden 10 »g EcoRI-geschnittene DNA aus AM80/5793 mit EcoRI-geschnittenen EMBL4-Armen ligiert und auf einem C600-Bakterienrasen ausplattiert. Etwa 500000 Plaques wurden erhalten, die statistisch gesehen das Genom der Kartoffel repräsentieren sollten. Nach dem Transfer dieser Plaques auf Nitrozellulose konnte durch Plaquehybridisierungstechniken gegen radioaktiv markierte wun1-cDNA der genomische Klon wun1-22 und wun1-85 identifiziert und gereinigt werden.

Die Isolierung von rekombinanter EMBL4-DNA aus wun1-22 und wun1-85 sowie deren Restriktionskartierung und Hybridisierung gegen radioaktive wun1-cDNA ergab die folgende Organisation.

Das wun1-cDNA-äquivalente Fragment in wun1-85 hat eine Größe von 4 Kb. Es entspricht damit exakt mit der in wun1-cDNA hybridisierenden Fragmentgröße EcoRI-verdauter DNA aus der haploiden Kartoffel. Aufgrund der asymmetrischen XhoI-Schnittstelle im 5′-Bereich des cDNA-Klons konnte die Orientierung des Gens in dem 4-Kb-Fragment ermittelt werden. Demnach liegt vor dem wun1-Gen ein etwa 1 Kb großer Promotorenbereich, während sich 3′ vom Gen ein etwa 2 Kb großer nicht homologer Teil befindet. Das zusätzlich in wun1-85 enthaltene 8 Kb große EcoRI-Fragment konnte nicht zur näheren Analyse des wun1-Gens herangezogen werden. Aufgrund des Totalverdaus der Kartoffel-DNA mit EcoRI ist es wahrscheinlich, daß bei der anschließenden Ligation zwei nicht zusammengehörige Fragmente in den EMBL4-Vektor gebracht wurden, die demnach nicht in funktioneller Beziehung zueinander stehen können.

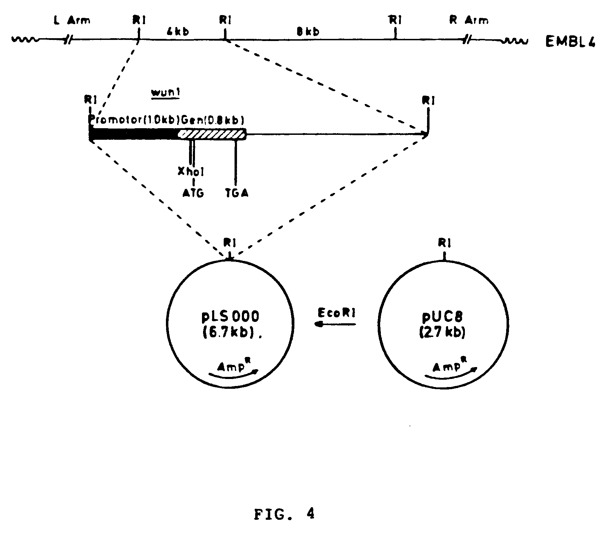

Fig. 4 zeigt die Anordnung von wun1 in dem genomischen Klon wun1-85. Auf dem 4 Kb großen EcoRI Fragment des genomischen Klons wun1-85 sind 1,0 Kb des wun1-Promotors, 0,8 Kb des wun1-Gens sowie 2,0 Kb des 3′-Endes vorhanden.

Da im Restriktionsverhalten keine Unterschiede zwischen wun1-85 und wun1-22 festgestellt wurden, wurde zur weiteren Analyse des 4 Kb-Fragments von wun1-85 in EcoRI-geschnittenen pUC8 ligiert (s. Fig. 4). Zur Sequenzierung des wun1-Promotors sowie des wun1-Gens erfolgte eine weitere Umklonierung des 4 Kb-Fragments in die EcoRI-Schnittstelle von M13mp18. Die Bestimmung seiner Orientierung wurde mittels Kontrollverdauungen an der asymmetrisch gelegenen XhoI-Schnittstelle durchgeführt. Die Klone 85*mp18 und 85mp18 repräsentieren beide Orientierungen des Fragments. Das Schneiden dieser Plasmide mit SphI und XbaI ermöglichte es, mit Hilfe der ExonukleaseIII beim Klon 85mp18 des 3′-Endes des wun1-Gens und beim Klon 85*mp18 das 5′-Ende des wun1-Gens sukzessive abzudauen.

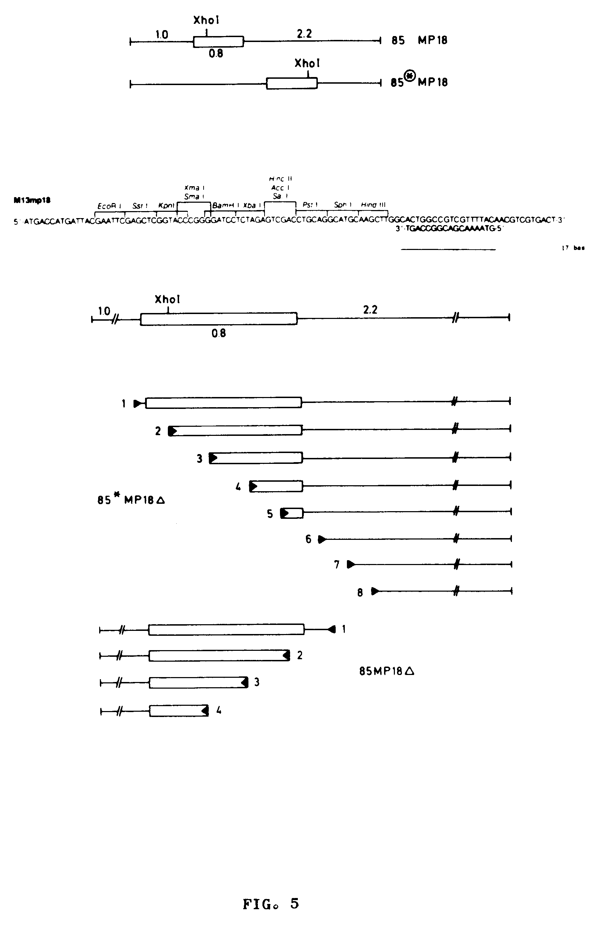

Fig. 5 zeigt das Ergebnis der Deletionsanalyse des Klons wun1-85 in schematischer Form.

Die resultierenden Deletionsklone mit unterschiedlichen Fragmentgrößen wurden für die Sequenzierung benutzt. Insgesamt gelang es, das gesamte wun1-Gen bidirektional zu sequenzieren, sowie zusätzlich etwa 400 bp des 3′-Endes unidirektional zu analysieren.

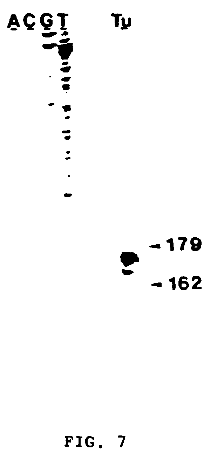

Fig. 6 zeigt die Nukleotidsequenz des wun1-Promotors und Gens aus wun1-85. CAAT-Box, TATA-Box und Poly-A-Signal sind herausgehoben, Transskriptionsstart und -stop durch Pfeile markiert. Zur Identifikation des genauen Transkriptionsstarts des wun1-Gens wurde die Methode der S1-Nuklease-Kartierung benutzt. Fig. 7 erläutert die Bestimmung der Größe des S1-geschützten Fragments des wun1-Gens. Die Hybridisierung des 5′ von der XhoI-Schnittstelle gelegenen Bereichs aus pLS000 mit wun1-mRNA führt zu einem 162-179 bp langen DNA-RNA-Hybrid (TU), das vor der einzelstrangspezifischen S1-Nuklease geschützt ist. Der tatsächliche Transkriptionsstart liegt also 162-197 bp 5′ von der XhoI Schnittstelle entfernt (A,C,G,T = Sequenzauftragung zur Größenbestimmung des DNA-Fragment).

Geht man von dem längsten erhaltenen Fragment aus, so beginnt der Transkriptionsstart 179 bp stromaufwärts von der XhoI-Stelle, d.h. mit der Sequenz ACCATAC. Diese Sequenz stimmt im zentralen Bereich (CAT) mit der von Joshi et al. (1987) ermittelten Konsensussequenz für Transkriptionsstart CTCATCA überein. Aus der Position des Transkriptionsstarts ergeben sich weitere Informationen (s. Fig. 6):
(1) In Position-33, gesehen vom Transkriptionsstart, ist eine TATA-Box CTATATATT zu finden, die gut mit der von Joshi et al. (1987) ermittelten Konsensussequenz TCACTATATATAG übereinstimmt.
(2) Die von Benoist et al., Nuc. Acids Res. 8, 127-142 (1980) beschriebene CAAT-Box im Bereich zwischen -60 und -80 ist im wun1-Promotor in Position -58 (CAAACT) zu finden.
(3) Der 5′-untranslatierte Bereich des wun1-Gens erstreckt sich über 217 bp und ist damit verhältnismäßig groß.
(4) Das wun1-mRNA kodierende Gen umfaßt 794 bp, was sehr genau der Größenbestimmung von wun1-mRNA aufgrund der "Northern-Blot"-Analysen entspricht.
(5) Dem cDNA-Klon wun1-25A2 fehlen 97 bp des 5′-untranslatierten Bereichs im wun1-Gen.
(6) Neben den bereits im cDNA-Klon ermittelten offenen Leserastern werden keine weiteren im 5′-untranslatierten Bereich des genomischen Klons gefunden.
(7) Das wun1-Gen verfügt nicht über Introns.

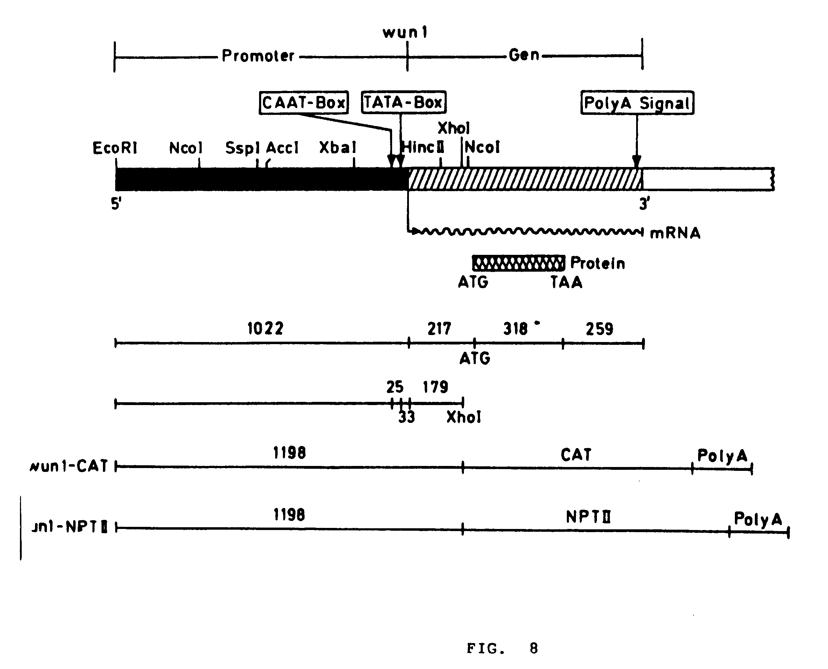

Fig. 8 zeigt die Anordnung und Position wichtiger Bereiche im wun1-Gen. Der wun1-Promotor ist schwarz markiert, das wun1-Gen schraffiert. Wichtige Erkennungssequenzen sind eingerahmt; Die mRNA ist durch eine geschlängelte Linie, der proteinkodierende Bereich durch Kreuze wiedergegeben. Die Größe der einzelnen Bereiche ist in Basenpaaren (bp) angegeben.

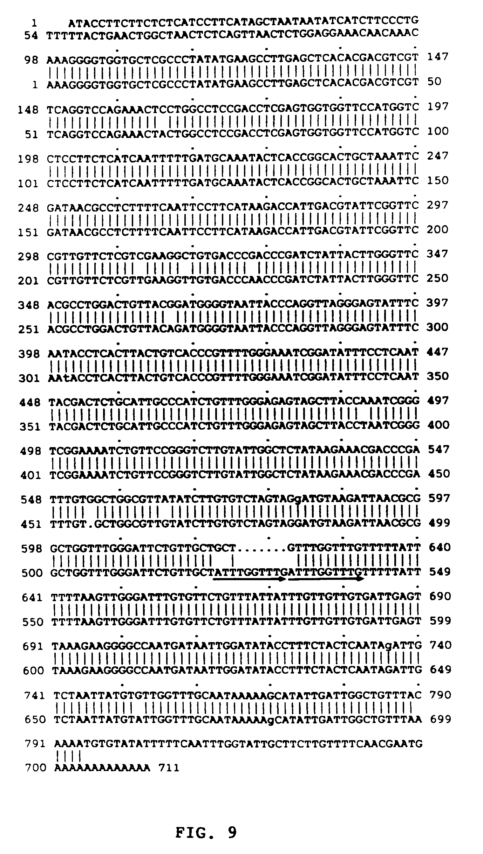

Fig. 9 zeigt einen Sequenzvergleich zwischen dem cDNA-Klon wun1-25A2 und dem genomischen Klon wun1-85. 2368 bp des genomischen Klons wun1-85 wurden mit 711 bp des cDNA-Klons wun1-25A2 verglichen. Homologe Basenpaarungen sind durch einen senkrechten Strich gekennzeichnet. Das Fehlen von Nukleotiden wird durch einen Punkt aufgezeigt. Die zwei Pfeile dokumentieren die Sequenz zweier 10 bp langer direkter Sequenzwiederholungen im cDNA-Klon.

Vergleichende Analyse zeigt, daß die Sequenz des genomischen Klons wun1-85 der haploiden Kartoffel AM80/5793 mit der Sequenz des cDNA-Klons wun1-25A2 der tetraploiden Kartoffel "Granola" zu 97 % homolog ist. Insgesamt konnten 11 Basenaustausche identifiziert werden, davon 5 im translatierten Bereich. Alle 5 Basenaustausche im translatierten Bereich führten zu keiner Veränderung der Aminosäuresequenz. Die zusätzlich sequenzierten 400 Nukleotide stromabwärts des Transkriptionsstops wiesen keine bemerkenswerten Bereiche auf.

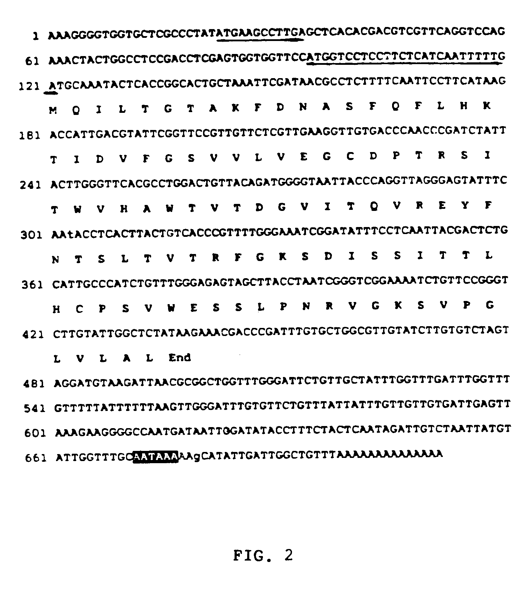

Das Gen codiert für ein 12000 Dalton großes Protein, dessen Größe auch in Hybrid-Released-Translation-Experimenten ungefähr bestätigt werden konnte. Computerauswertungen bezüglich der Aminosäurezusammensetzung des wun1-Proteins ergaben, daß es sich um ein sehr hydrophiles Protein handelt. Die Aminosäuresequenz ergibt sich aus Fig. 2 und 6.

Die Größe des wun1-Proteins, seine hydrophilen Eigenschaften, die Verwundungsinduzierbarkeit in verschiedenen Geweben und die Induzierbarket durch Pathogene lassen auf seine Zugehörigkeit zu den PR-Proteinen schließen. Mit dem Namen PR- oder "Pathogenesis-related"-Protein werden Proteine verschiedener Pflanzen zusammengefaßt, die unter anderem durch pathogenen Befall induzierbar sind und in einigen Fällen Chitinase- bzw. Glucanase-Aktivität aufweisen, also die Aktivität von Enzymen, die Zellwände von Pilzen zerstören können.

Der vollständige genomische wun1-Klon inclusive wun1-Promotor und wun1-Gen wurde unter Verwendung des Agrobakterium-Transformationssystems in transgenen Tabakpflanzen getestet. Eine Expressionsstudie war auf mRNA-Ebene möglich, da Tabak-mRNA nur sehr schwach mit wun1 kreuzhybridisiert und zusätzlich über keine wun1-homologen 5′-Enden verfügt, wie aus S1-Nuklease-Kartierungen zu entnehmen war. Tatsächlich wurde in transgenen Tabakpflanzen in verwundeten Blättern hohe Mengen an etwa 800 bp großer wun1-mRNA nachgewiesen, was einerseits einen aktiven wun1-Promotor dokumentiert, andererseits auch die Verwendung des richtigen Transkriptionsstartpunktes zeigt.

Ein 4 Kb-Fragment aus pLS000, welches den wun1-Promotor, das vollständige wun1-Gen, sowie 2 Kb des 3′-Bereichs des Gens enthält (wun1-wun-1) wurden über die EcoRI-Schnittstellen in den für die Agrobacterium tumefaciens(At)Transformation benötigten Mobilisierungsvektor pMPK110 kloniert (pLS001). Nach erfolgtem Transfer in das At3850ₖₘ, wurde die Tabaklinie Wisconsin 38 (W38) mit diesem Agrobakterium über Blattstücke transformiert. Die unter Kanamycinselektion nach etwa 3 Wochen erscheinenden kleinen Kalli wurden durch die Verwendung eines Sproßinduktionsmediums zur vollständigen Pflanze (LS1) regeneriert.

Neben der Fähigkeit, auf Kanamycin wachsen zu können, besitzen mit 3850_{Km} transformierte Pflanzen die Fähigkeit zur Synthese von Nopalin, eine Eigenschaft, die sie von untransformierten Pflanzen unterscheidet (Zambryski et al, EMBO J. 1 147-152 (1985)). Wun1-wun1 transformierte Pflanzen, hier LS1-Pflanzen genannt (4= LS1-4; 6=LS1-6), zeichneten sich durch hohe Nopalinmengen aus. In untransformierten Pflanzen war dagegen kein Nopalin nachweisbar. Kanamycin-resistente und Nopalin-positive LS1-Pflanzen wurden in das Gewächshaus transferiert und auf DNA- und RNA-Ebene analysiert.

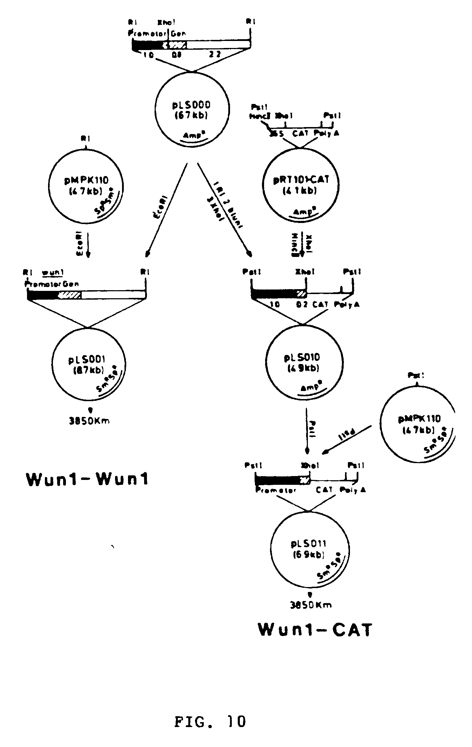

Fig. 10 zeigt die Konstruktion von wun1-wun1- und wun1-CAT-Fusionen, die für Expressionsuntersuchungen auf Basis von transienten Expressionen und stabilen Transformationen verwandt wurden.
(1) Herstellung des Plasmids pLS001:
   Ausgehend von Plasmid pLS000 (s. Fig. 4) wurde das 4 Kb-Fragment, das den wun1-Promotor, das wun1-Gen sowie 2 Kb des 3′-Endes enthält, über die EcoRI-Schnittstelle in den Vektor pMPK110 kloniert (pLS001). Dieses pMPK110 Derivat erlaubt den Transfer der wun1-wun1-Konstruktion ins Agrobakterium 3850_{Km}.
(2) Herstellung des Plasmids pLS011:
   Der wun1-Promotor sowie 179 BP des 5′-untranslierten wun1-Genbereichs wurden über die XhoI Schnittstelle mit dem CAT-Gen inclusive 3′-Ende fusioniert (pLS010). Eine Umklonierung über PstI in den Vektor pMPK110 (pLS011) hat den Vorteil, daß dieses Plasmid einerseits für transiente Studien in Protoplasten benutzt werden kann und andererseits auch als Mobilisierungsplasmid zum Transfer der wun1-CAT-Konstruktion in das Agrobakterium 3850_{Km} fungiert.

Die "Southern-Blot"-Analyse EcoRI verdauter DNA aus transgenen LS1-Tabakpflanzen, sowie untransformierter W38-Tabakpflanzen ergab bei Verwendung einer ³²P-radioaktiv markierten wun1-cDNA Probe sowohl in den untransformierten als auch in den tranformierten Pflanzen DNAs eine positive Hybridisierung. In beiden Fällen war eine starke und eine schwache Bande erkennbar. In den transgenen LS1-Pflanzen erschien jedoch zusätzlich eine 4 Kb große Bande, die in ihrer Größe exakt der Größe des EcoRI-Inserts aus pLS000 entsprach. Durch verleichende Auftragung verschiedener DNA-Mengen konnte eine 2 bis 5fache Kopienzahl von wun1-wun1 in allen getesteten LS1-Pflanzen ermittelt werden. In einigen LS1-Pflanzen wurden zusätzliche Banden im hochmolekularen Bereich gefunden, die vermutlich auf rearrangierte DNA oder unverdaute DNA zurückzuführen sind.

Eine funktionelle Analyse der wun1-wun1-DNA positiver LS1-Pflanzen war auf RNA-Ebene möglich. Sowohl in verwundeten als auch in unverwundeten untransformierten Tabakpflanzen war nur eine sehr schwache Kreuzhybridisierung gegen eine wun1-cDNA-Probe vorhanden. Zusätzlich wurde isolierte RNA aus untransformierten Tabakpflanzen gegen den 5′-Bereich des wun1-Gens, das den 5′-untranslierten Bereich und den wun1-Promotor enthält (analog zum Fragment zur Transkriptionsstartbestimmung des wun1-Gens) über S1-Analyse auf homologe Bereiche hin getestet. Es zeigte sich, daß weder in unverwundeten noch in verwundeten untransformierten Tabakblättern oder -stengeln wun1-homologe Sequenzen vorhanden waren.

Von 30 Nopalin-positiven LS1-Pflanzen, die auf RNA-Ebene untersucht wurden, zeigten 5 Pflanzen in "Northern-Blot"-Experimenten hohe Mengen an 800 bp großen mit wun1 hybridisierenden mRNA's. Deren S1-Analyse führte zum Nachweis zweier gleich großer DNA-Fragmente, die in ihrer Größe den Fragmenten aus verwundeten Kartoffelblättern entsprachen. Dies war die Bestätigung, daß es sich hier um wun1-homologe mRNA handelt. Mehrere Hinweise sprechen dafür, daß wun1-homologe mRNA auch als Antwort auf hohen TMV-Befall gebildet wird.

Ein Ziel der Erfindung ist die Konstruktion und Isolierung von Genen, die in der Lage sind, den Aufforderungen entsprechend fusionierte Proteine zu exprimieren. Hierzu ist erforderlich, daß der wun1- Promotor allein für die Expression des nachgeschalteten Strukturgens verantwortlich ist, was nicht immer der Fall sein muß (Bsp. Proteinase-Inhibitor II).

Es wurden transskriptionelle Fusionen, bestehend aus dem wun1-Promotor und 178 bp des 5′-untranslierten Gens, mit verschiedenen Strukturgenen fusioniert (wun1-CAT, wun1-NPT, wun1-GUS), deren Nachweis über radioaktive oder fluorimetrische Methoden möglich war. (GUS steht für ß-Glucuronidase (Jefferson et al, Proc. Nat. Acad. Sci. 83, 8447-8451 (1987)).

Die Konstruktion wun1-CAT wurde in Kartoffelprotoplastern auf ihre transiente Funktionalität getestet. Diese Methode ermöglicht es, innerhalb von wenigen Tagen Promotoraktivitäten zu analysieren, indem hochgereinigte Plasmid-DNA, die die Konstruktionen enthält, mittels bekannter Methoden in Protoplasten transferiert wird, dort stabil vorliegt und auch abgelesen werden kann. Voraussetzung für eine Expression ist, daß der Promotor funktionell ist, und daß die induzierenden Faktoren in solchermaßen behandelten Protoplasten vorhanden sind.

Bei diesen Tests wurde der wun1-Promotor inclusive 179 bp des 5′-untranslierten Bereichs transkriptionell mit dem Chloramphenicolacetyltransferase-Gen (CAT) fusioniert. Die Konstruktion der wun1-CAT-Fusion ist in Fig. 10 dargestellt.

Der im pRT101-CAT enthaltene 35S-Promotor wurde über die Schnittstellen HincII/XhoI entfernt und statt dessen der 1,0 Kb große wun1-Promotor mitsamt 179 bp des 5′-untranslatierten Bereichs über eine aufgefüllte EcoRI Schnittstelle und XhoI eingesetzt. Dieses chimäre Gen wun1-CAT konnte über die PstI-Schnittstellen in den Vektor pMPK110 gebracht werden (pLS011).

In den für transiente Tests verwendeten Kartoffelzellsuspensionen sowie daraus gewonnene Protoplasten konnten hohe Mengen an endogener wun1-mRNA festgestellt werden. Es wird angenommen, daß die Flüssigkultivierung von Zellen und vor allem die Protoplastierung einen hohen Streß auf die Zelle ausüben, was sich analog dem Verhalten in der Kartoffelknolle in einer hohen Expression von wun1-mRNA äußert.

Zur transienten Analyse der wun1-CAT Konstruktion wurden jeweils 1 Million Protoplasten aus einer Suspensionskultur aus Kartoffelstengeln der Varietät Datura (D12) isoliert und nach der CaCl₂/PEG-Methode mit 20 »g pLS011-DNA transformiert.

Die CAT-Analyse solcher mit pLS011-DNA-transformierten Protoplasten ergab hohe CAT-Aktivitäten. Ein paralleler Versuchsansatz mit einer CAMV-35S-CAT-Konstruktion (35S-Promotor aus dem Cauliflower-Mosaikvirus (CAMV)) (pRT101-CAT) brachte in etwa vergleichbare CAT-Aktivität, während in einem Kontrollansatz ohne Zusatz von DNA keine CAT-Aktivität zu erkennen war. Dies bedeutet, daß in einem transienten Kartoffelprotoplastensystem die Aktivität des wun1-Promotors mit der des 35S-Promotors vergleichbar ist und somit als sehr hoch einzustufen ist. Der CAMV-Promotor gilt als stärkster bisher bekannter Promotor in Pflanzen.

Das schon im transienten System benutzte Plasmid pLS011 (wun1-CAT) konnte weiterhin zur Transformation von At 3850_{Km} benutzt werden. 3850_{KM}::pLS011 Agrobakterien wurden über die Methode der Blattscheibeninfektion zur Transformation der Tabaksorte Samsum NN (SNN) benutzt und entstehende Kalli zur Pflanze (LS2) regeneriert.

Durch die Verwendung dieses Tabakmosaikvirus(TMV)resistenten Tabaksorte sollte die (vermutete) Induzierbarkeit von wun1 durch TMV umgangen werden. Im Nopalin-Test positive und kanamycinresistente LS2-Pflanzen wurden auf DNA-Ebene und auf das Vorhandensein von CAT-Aktivität getestet. PstI geschnittene DNA aus Wun1-CAT transformierten Pflanzen wiesen ein 2,2 Kb großes, gegen CAT-CDNA hybridisierendes Fragment auf. Dieses entsprach genau der zu erwartenden Fragmentgröße von wun1-CAT. In untransformierten Pflanzen war keine Hybridisierung zu erkennen. Durch vergleichende DNA-Auftragungen konnte in allen positiven Pflanzen 2 bis 4 Kopien pro haploidem Genom ermittelt werden.

Die funktionelle Analyse von LS2-Pflanzen erstreckte sich auf die Messung von CAT-Aktivitäten in Gewächshauspflanzen und auf in Sterilkultur gehaltene Pflanzen der F1-Generation. Im Gewächshaus angezogene LS2-Pflanzen zeigten in ihren unverwundeten Blättern eine im Vergleich zu der Hintergrundaktivität untransformierter Tabakblätter leicht erhöhte CAT-Aktivität. In verwundeten Blättern konnte im Vergleich zu unverwundeten Blättern eine etwa 4fach höhere CAT-Aktivität nachgewiesen werden. Gab man jedoch in den Verwundungsansatz zusätzlich einen Überstand aus abgekochtem Kartoffelknollenextrakt, so war der Anstieg 6fach höher als im unverwundeten Zustand. Dieser Effekt war unabhängig vom Zustand der Knollen vor der Extraktion (unverwundete oder verwundete Knollen bzw. frisch aus dem Boden entnommene (ungelagert) oder gelagerte Knollen). Jeder Extrakt hatte den gleichen induzierenden Effekt, was auf einen im Extrakt vorhandenen Induktor hindeutet.

Die Ursache der relativ niedrigen Induzierbarkeit von wun1-CAT in transgenen Tabakpflanzen, ist in der Aktivität in unverwundeten Blättern zu sehen. Um mögliche gewächshausbedingte Ursachen auszuschalten, wurde der Samen geselbsteter LS2-Pflanzen geerntet, und unter sterilen Bedingungen wieder ausgekeimt. In jungen Blättern kanamycinresistenter Pflanzen war im unverwundeten Zustand eine sehr schwache CAT-Aktivität meßbar, die sich von der CAT-Aktivität untransformierter Blätter wenig unterschied. In verwundeten Blättern stieg die CAT-Aktivität mindestens um den Faktor 40 an. Die Zugabe von abgekochten Überständen aus homogenisierten Kartoffelknollen in den Verwundungsansatz führte sogar zu einer 60fach höheren Expression.

Zusätzlich war zu beobachten, daß mit zunehmendem Alter der Pflanze die Aktivität des wun1-Promotors in den Blättern immer constitutiver wurde, wobei die Expressionsstärke die einer transgenen CAMV-CAT-Pflanze glich. Möglicherweise wirken hier Vorgänge der Seneszenz ebenfalls induzierend.

Weiter wurde mit Hilfe des transienten Systems getestet, in welchen Pflanzenspecies der wun1-Promotor aktiv ist, und dementsprechend verwendbar ist. Es stellte sich heraus, daß dieser Promotor nicht nur in Kartoffeln-, Tabak- und Petersilienprotoplasten aktiv ist, sondern auch in Reisprotoplasten eine Expressionsstärke zeigt, die der Aktivität eines homologen Systems entspricht.

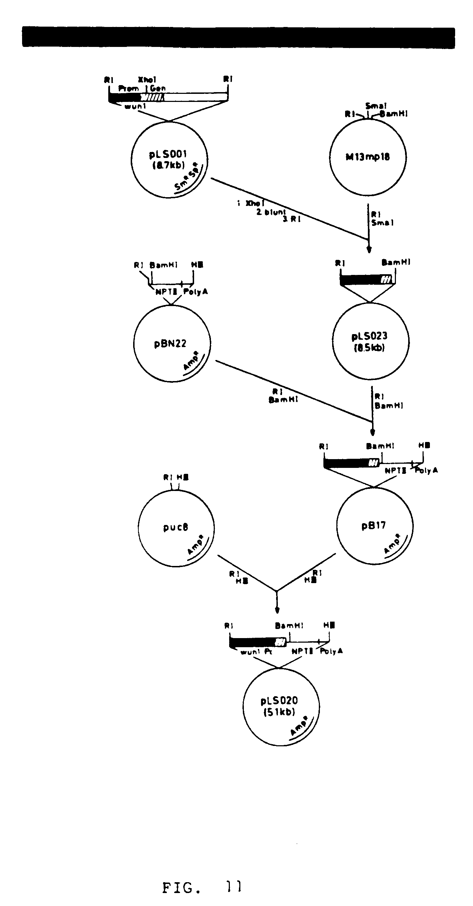

Fig. 11 zeigt die Herstellung von wun1-NPTII Konstruktionen. Ausgehend von Plasmid pLS001 wurde der wun1-Promotor inclusive 179 bp des 5′-untranslatierten Bereichs über EcoRI-XhoI-blunt vor ein NPT-II Gen (Neomycinphosphotransferase-II) kloniert. Zur besseren Handhabung für transiente Experimente wurde die wun1-NPT-II Fusion in einen hochreplizierenden Vektor umkloniert (pLS 020).

Unter Verwendung der wun1-NPTII Konstruktion wurde in Tabak und in Petersilieprotoplasten NPT-II Aktivität gefunden. Interessanterweise konnte wun1-abhängige NPT-II Aktivität auch in Reisprotoplasten (Oryza sativa japonica c.v. Taipai) nachgewiesen werden, die in ihrer Intensität mit der wun1-NPTII Aktivität in Kartoffelprotoplasten vergleichbar war. Vergleichende transiente Expression von wun1-NPTII und NOS-NPTII (pGV1103) in Reis-Protoplasten zeigten ebenfalls ähnlich hohe Werte. In diesem Zusammenhang wird auch noch einaml auf die CAMV-35S-CAT ähnliche transiente Expression in Kartoffelprotoplasten hingewiesen. Insgesamt belegen diese Studien, daß der wun1-Promotor in homologen wie auch im heterologen System über eine teilweise sehr hohe Aktivität verfügt. Weder in Petersilie- noch in Tabaknoch in Reisprotoplasten konnte mit wun1-hybridisierende mRNA gefunden werden. Es müssen also den wun1-Promotor stimulierende Faktoren angenommen werden, die in allen getesteten Pflanzenspezies gleichermaßen vorkommen.

Insgesamt gesehen sind in dem 5'-Bereich von wun1 alle wichtigen DNA-Bereiche vorhanden, um ein dahinter fusioniertes Gen in hohen Mengen und verwundungsinduzierbar exprimieren zu können.

Zusätzlich läßt die Funktionalität von wun1-CAT bzw. wun1-NPTII in diversen transienten Systemen (Kartoffel, Petersilie, Tabak, Reis) wie auch im stabil transformierten Tabak auf jeweils ähnliche molekulare Hintergründe schließen.

Im Rahmen der vorliegenden Erfindung durchgeführte Versuche ergaben ferner, daß es vorteilhaft sein kann, den erfindungsgemäßen wun1-Promotor mit anderen Promotoren oder Verstärkerelementen zu fusionieren.

Als Beispiel seien die Fusionierung des wun1-Promotors mit dem bekannten Mannopin-Synthase-(MAS)-Promotor von der Agrobacterium tumefaciens TR-DNA erwähnt.

Die Isolierung dieses MAS-Promotors von der Agrobacterium tumefaciens TR-DNA ist aus einer Arbeit von J. Velten und J. Schell "Selection-expression vectors for use in genetic transformation of higher plants", veröffentlicht in Nucl. Acids Res., 13:6981-6997 bekannt. Aus dieser Literaturstelle ist es auch bekannt, daß dieser Promotor für die Expression der Mannopine-Synthase verantwortlich ist und folgende Charakteristika aufweist:
1. Er ist bidirektional aktiv, d. h. fusioniert man an beide Seiten Strukturgene, so werden beide in ähnlicher Regulationsweise und Intensität abgelesen. Verwiesen wird in diesem Zusammenhang auch auf die Literaturstelle: Langridge, W.H.R., Fitzgerald, K.J., Koncz, C., Schell, J. und Szalay, A.A. (1989). Dual promoter of Agrobacterium tumefaciens mannopine synthase genes is regulated by plant growth hormones. Proc. Natl. Acad. Sci. USA, 86:3219-3223.
   Die Orientierung des MAS-Promotors hat man mit 1' und 2' festgelegt.
2. In transgenen Pflanzen ist der MAS-Promotor verwundungsinduzierbar. Eine Fusion des MAS2' mit dem Referenzgen beta-galactosidase (beta-gal) führt in verwundeten transgenen Tabakpflanzen zu einer höheren beta-gal-Aktivität als in unverwundeten.
3. Die Aktivitiät des MAS2'-Promotors ist hauptsächlich in den Blattvenen, also im Leitbündelsystem des Blattes zu finden. Zu 2. und 3. wird verwiesen auf: Teeri, T.H., Lehväslaiho, H., Franck, M., Uotila, J., Heino, P., Palva, E.T., Van Montagu, M. und Herrera-Estrella, L. (1989). "Gene fusions to lacZ reveal new expression patterns of chimeric genes in gransgenic plants". EMBO, 8:343-350.
4. Die Eigenschaften des MAS1'-Promotors sind bisher nicht so intensiv untersucht worden. Es ist aber bekannt, daß der MAS1'-Promotor etwa 4-7fach schwächer ist als der MAS2'-Promotor, ansonsten aber über ähnliche Eigenschaften verfügt.

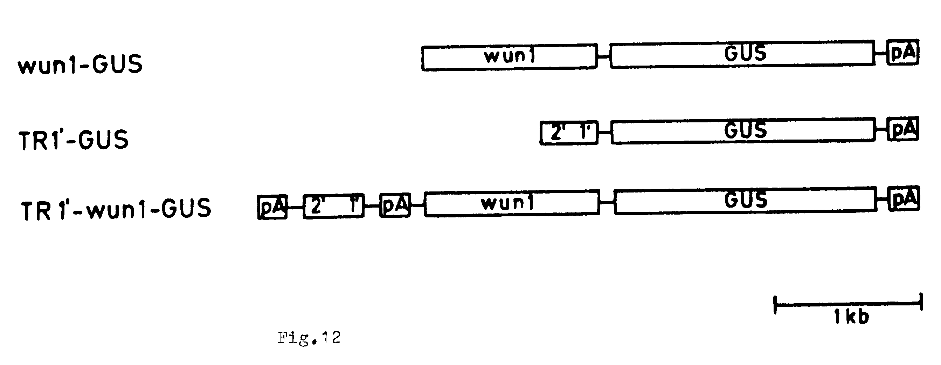

Im Rahmen von Versuchen mit dem Ziel einer qualitativen und quantitativen Optimierung des wun1-Promotors wurde der MAS-Promotor an den wun1-Promotor fusioniert, und zwar in der Orientierung MAS1'-wun1 (Fig. 12). Zum Nachweis der Promotoraktivitiät wurde das Markierungsgen GUS (beta-Glucoronidase) verwandt (MAS1'-wun1-GUS). Mittels des Agrobacterium tumefaciens Transformationssystems wurde diese Konstruktion in Tabak transformiert und analysiert. Folgende Eigenschaften zeichnen diese Tabakpflanzen im Vergleich zu wun1-GUS transgenen Tabakpflanzen aus:
1. Die Gewebespezifität hat sich geändert.
   In wun1-GUS transgenem Tabak findet man GUS-Aktivität hauptsächlich in der Epidermis von Blättern und Stengeln. In MAS1'-wun1-GUS transgenem Tabak ist die GUS-Aktivität hauptsächlich im Leitbündelsystem lokalisiert. Somit besteht die Möglichkeit, je nach wissenschaftlicher Fragestellung, Genprodukte an verschiedene Stellen des Gewebes dirigieren zu können. Das Wundinduktionsverhalten von MAS1'-wun1-GUS transgenen Tabakpflanzen ist dagegen mit dem Verhalten von wun1-GUS transgenen Tabakpflanzen vergleichbar.
2. Insgesamt ist die Aktivität des MAS1'-wun1-Promotors in transgenen Tabakblättern und -stengeln etwa 10fach höher als die Aktivität des wun1-Promotors. Somit zählt der MAS1'-wun1-Promotor zu den stärksten Promotoren, die es für eine Expression in Pflanzen gibt.
3. Während Promotorenaktivität in transgenen dicotylen Pflanzen heutzutage kaum ein Problem darstellt, gibt es bisher wenige Promotoren, die hinreichend aktiv sind in monocotylen Pflanzen (zu denen die wirtschaftlich wichtigen Getreidepflanzen gehören). In transienten Expressionsstudien in Reis-Protoplasten (sie dienen als Ersatz für Studien in transgenen Reispflanzen, die nur sehr schwer zu erhalten sind) zeichnet sich der MAS1'-wun1-Promotor durch eine etwa 30fach höhere Aktivität aus als der wun1-Promotor. Dies ist umso bemerkenswerter, weil selbst der in dicotylen Pflanzen so starke 35S-Promotor hier 30fach schwächer als der MAS1'-wun1-Promotor ist.

Somit läßt sich beispielsweise der erfindungsgemäße wun1-Promotor durch Fusion mit dem MAS1'-Promotor in folgenden Eigenschaften verändern bzw. optimieren:
(a) Die spezifische Aktivität des wun1-Promotors in der Blatt- bzw. Stengel-Epidermis von transgenem Tabak wird durch die Fusion mit dem MAS1'-Promotor in das Leitbündelsystem verlagert;
(b) Die Verwundungsinduzierbarkeit des wun1-Promotors wird durch die Fusion nicht geändert;
(c) Der MAS1'-wun1-Promotor ist in transgenen Tabakblättern und -stengeln etwa 10fach stärker als der wun1-Promotor allein;
(d) Der MAS1'-wun1-Promotor ist in transienten Experimenten mit einem Vertreter der monocotylen Pflanzen (Reis) etwa 30fach stärker als der wun1-Promotor.

Erfindungsgemäß läßt sich ferner z.B. die Expression des wun1-Promotors durch Fusion desselben mit einem Verstärkerelement des CamV-35S-Promotors verstärken. So führt beispielsweise die Fusion des Verstärkerelementes des CamV-35S-Promotors an das 5'-Ende des wun1-Promotors zu einer verstärkten wun1-Promotoraktivität in transgenem Tabak und Kartoffel.

Fig. 12 zeigt die Organisation der Klone wun1-GUS, MAS1'-GUS und MAS1'-wun1GUS.

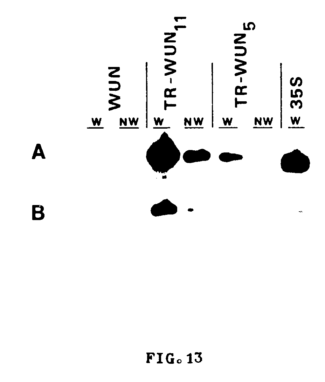

Fig. 13 zeigt die Northern-Blot-Analyse von verwundeten und unverwundeten transgenen Tabakblättern. Unverwundete (NW) bzw. verwundete (W) Blätter der transgenen Tabakpflanzen wun1-GUS, MAS1'-wun1-GUS Nr. 5 und Nr. 11 und 35S-GUS wurden zur Isolierung von RNA benutzt. Je 50 »g Gesamt-RNA wurde auf einem 1,2%igen Formaldehyd-Gel gelelektrophoretisch getrennt, auf eine Nylonmembran transferiert, gegen eine radioaktiv markierte GUS-DNA Probe hybridisiert, und spezifische Bindungen auf einem Autoradiogramm sichtbar gemacht. (A) zeigt eine 2 Tage alte Exposition, (B) eine 6 Stunden alte Exposition.

Die nachstehenden Beispiele dienen der näheren Erläuterung der Erfindung:

### Beispiele 1-8

### Materialien

### Medien

### Kulturmedien für Bakterien:

Zur Anzucht von Bakterien verwendete Medien wurden nach den Angaben von Maniatis et al., Molecular Cloning: A Laboratory Manual, Gold Spring Harbour Laboratory, Gold Spring, Harbour, New York (1982) hergestellt:

### Pflanzenmedien:

Die benutzten Medien leiten sich von dem von Murashige und Skoog, Physiol. Plan. 15, 473-497 (1962), angegebenen Medien (MS) ab.
- 3MS :: MS + 3 % Saccharose
- 3MSC :: MS + 3 % Saccharose, 500 »g/ml Claforan
- MSC10 :: MS + 2 % Saccharose, 500 »g/ml Claforan,
0,2 »g/ml NAA, 1»g/ml BAP
100 »g/ml Kanamycinsulfat
- MSC15 :: MS + 2 % Saccharose, 500 »g/ml Claforan,
100 »g/ml Kanamycinsulfat
- MS15 :: MS + 2 % Saccharose, 100 »g/ml Kanamycinsulfat
Für festes Medium wurden zusätzlich 8g/l Bacto-Agar zugegeben.

### Stämme und Vektoren

E. coli-Stämme:
- BMH 71-18:: (1ac-proAB), thi, supE; F′(laci^{q}, ZdeltaM15, proA⁺B⁺) (Messing et al., Proc. Nat. Acad. Sci. 74, 3642-3646 (1977))
- C 600 :: = CR34 (Maniatis et al. 1982)
- GJ23 :: AB1157 (R64drd11) (pGJ28) (Van Haute et al., EMBO J. 2, 411-418, (1983))
- Agrobakterien-Stämme :: C58CIpGV3850_{KM} (Zambryski et al., EMBO J. 1, 147-152, (1983))
- Plasmide :: pUC8 (Vieira und Messing, Gene 19, 259-268 (1982))
pMPK110 (Eckes et al., Mol. Gen. Genet. 205, 14-22 (1986))
pRT101-cat (Pröls et al., Plant Cell Reports, im Druck (1988))
pGV1103 (Hain et al., Mol. Gen. Genet. 199, 166-168 (1985))
- Phagen :: EMBL4 (Frischauf et al., J. Mol. Biol. 170, 827-842 (1983))
M13mp18 (Yanisch-Perron et al., Gene 33, 103-119, (1985))
M13mp19 (Yanisch-Perron et al., 1985)
- Pflanzen:: Solanum tuberosum AM 80/5793 (haploid)
Berolina (tetraploid)
Datura (D12)
Granola
Nicotinia tabacum Wisconsin 38 (W38)
Samsum NN (SNN)
Oryza sativa japonica c.v. Taipai

Alle molekularbiologischen Standard-Methoden, wie z.B. Restriktionsanalyse, Plasmidisolierung, Minipräparation von Plasmid-DNA, Transformation von Bakterien usw. sind, sofern nicht anders angegeben, wie bei Maniatis et al. (1982) beschrieben, durchgeführt worden.

### Verwundetes Pflanzengewebe

Kartoffelknollenmaterial wurde in 3mm dicke Scheiben geschnitten und in einer Phosphatlösung (20 mM Phosphatpuffer, pH 7,0, mit Chloramphenicol (50 »g/ml)) 18 h lang bei 28°C unter Lichtausschluß inkubiert. Blatt-, Stamm- und Wurzelmaterial wurde in kleine Stücke zerschnitten und unter den gleichen Bedingungen inkubiert. Nach abgeschlossener Inkubation wurde das Material entweder direkt aufgearbeitet oder bei -70°C gelagert.

### Unverwundetes Pflanzengewebe

Blatt-, Stamm-, Wurzel- und Knollenmaterial wurde sofort nach seiner Entfernung von der lebenden Pflanze aufgearbeitet oder in flüssigem Stickstoff eingefroren und bei -70°C gelagert.

### Aerobe Verwendung von Kartoffelknollen

Behandlung wie in "Verwundung" beschrieben, wobei während der Inkubation die Knollenscheiben nur leicht mit dem Phosphatpuffer benetzt waren. Nach der Inkubation war die Knollenoberfläche angebräunt.

### Anaerobe Verwendung von Kartoffelknollen

Behandlung wie in "Verwendung" beschrieben, wobei während der Inkubation die Knollenscheiben vollständig in Phosphatpuffer eingetaucht waren. Nach der Inkubation war die Knollenoberfläche weiß-gelblich hell.

### Expression von wun1-mRNA in pathogenbefallenen Kartoffelblättern

Blätter der Sorte "Datura" wurden an ihrem Blattstiel abgeschnitten und mit dem Stiel in Wasser gestellt. Anschließend wurden diese Blätter mit einer Phytophtorasporen-Suspension besprüht, um dann zu verschiedenen Zeiten von je einem Blatt die Gesamt-RNA zu isolieren und über einen "Northern-Slotblot" zu untersuchen. Drei verschiedene Ansätze wurden verwendet:
- Wasser, das die Sporen eines in Bezug auf Datura kompatiblen Phytophtora infestans-Stammes PJ1 enthält;
- Wasser, mit Sporen des in Bezug auf Datura inkompatiblen Phytophtora infestans-Stammes PJ4;
- Wasser, ohne den Zusatz von Sporen (Kontrolle)
Die densitometrischen Auswertungen der wun1-Hybridisierungsstärke ergab bereits zwei Stunden nach Behandlung der Blätter mit Wasser und Sporen der inkompatiblen Phytophtoraspezies einen 4-fachen Anstieg der wun1-mRNA Menge. Von der 4-ten bis 8-ten Stunde blieb die Expressionsmenge ungefähr gleich, bevor sie dann bis zur 30-sten Stunde sukzessive auf den 1,5 bis 2-fachen Wert zurückfiel. Das gleiche Expressionsverhalten zeigte wun1-mRNA auch bei der Verwendung von Wasser ohne Sporen (Kontrolle).

Bei dem analogen Versuchsansatz mit Wasser und kompatiblen Phytophtorasporen war 2 h nach Befall ein 4-facher wun1-mRNA Anstieg zu beobachten, der sich bis zur 10-ten h nur leicht reduzierte. Spätestens ab der 16-ten h erhöhte sich jedoch die Expression um das 6-fache des Kontrollwertes, um nach konstanter Steigerung nach 30 h den Faktor 9 zu erreichen.

### Verwundung unter Zusatz von Kartoffelextrakt

Kartoffelknollen wurden ohne Zusatz von Puffern bei 4°C homogenisiert und 10 Minuten bei 10000 upm im SS34-Rotor zentrifugiert. Der Überstand wurde 10 Minuten gekocht und nochmals zentrifugiert. Der klare Überstand wurde im Verhältnis 1:10 zu 20mM Phosphatpuffer, pH 7,0 addiert und das zu testende Gewebe unter den normalen Bedingungen darin inkubiert.

### Beispiel 1

### RNA-Isolierung

Die Isolierung von RNA aus verschiedenen Organen der Kartoffel und des Tabaks wurden wie bei Logemann et al. in Anal. Biochem., 163:16-20 (1987) beschrieben durchgeführt.

Die selektive Anreicherung von PolyA⁺RNA erfolgte mittels mAP-Papiers (Werner et al., Analytical Biochem. 141, 329-336 (1984).

### "Northern-Blot"-Analyse

Die RNA wurde auf einem 1,5 %-igen Formaldehyd-Agarose-Gel elektrophoretisch aufgetrennt (Lehrach et al., Biochemistry 16, 4743, (1977)). Wie bei Willmitzer et al., EMBO J. 1, 139-146, (1982) beschrieben, wurde anschließend die RNA auf Nitrozellulose transferiert, fixiert und gegen ³²P*radioaktiv markierte cDNA hybridisiert, gewaschen und exponiert.

### DNA-Isolierung

Nukleäre DNA wurde nach der Methode von Bedbrook, PMB Newsletter II, 24 (1981) aus Kartoffelblättern gewonnen und für die Klonierung in LambdaPhagen EMBL4 verwandt. DNA aus transformiertem Gewebe wurde nach dem kombinierten Aufbruch mit Triton X 100, SDS, und Proteinase K (Wassenegger, Dissertation, Köln (1988)) gereinigt.

### "Southern-Blot"-Analyse

DNA wurde auf 0,8 bis 1,2 %-igen Agarosegelen elektrophoretisch getrennt, auf Nitrocellulose transferiert und fixiert (Southern, J. Mol. Biol. 98, 503-517 (1975)), sowie hybridisiert und gewaschen, wie bei Willmitzer et al. (1982) beschrieben.

### "Run-off"-Experimente

Eine Methode, die relativ genau zwischen diesen beiden Regulationstypen unterscheidet, ist die "Run-Off"-Methode. Hier werden Zellkerne aus verwundeten und unverwundeten Knollen isoliert, von ihnen neu synthetisierte mRNA radioaktiv puls-markiert und gegen Einzelstrang-DNA der einzelnen Klone hybridisiert. In Zellkernen aus unverwundeten Klonen wurde keine wun1-mRNA produziert, dagegen in Zellkernen aus verwundeten Knollen in hohen Mengen.

Die Isolierung von Zellkernen aus Kartoffelknollen, sowie die anschließende in-vitro-Transkription wurde nach Willmitzer et al., Nucl. Acids Res. 9, 19 (1981) durchgeführt. Die auf diese Weise erhaltenen radioaktiv markierten RNA-Transkripte wurden gegen bidirektional in M13 klonierte cDNA-Klone hybridisiert.

### Proteinisolierung und Auftrennung

Die Isolierung von Proteinen aus Kartoffelknollen sowie deren ein- und zweidimensionale gelelektrophoretische Auftrennung ist bei Mayer et al., Plant Cell Reports 6, 77-81 (1987) beschrieben.

### Beispiel 2

### "Hybrid-released-translation"-Experimente

Das 800 bp Fragment des cDNA-Klons wun1-25A2 und das 700 bp Fragment des cDNA-Klons wun2-29C12 wurden über die PstI Schnittstelle in M13mp18 kloniert. Die Orientierung der Insertionen in den M13-Klonen wurde durch Komplementations-Analysen (Messing, in: Genetic Engineering of Plants, (1983) Plenum Press, New York, 1983) bestimmt. Die Identifizierung der M13-Klone mit dem kodogenen Strang erfolgte durch ein positives Hybridisierungsverhalten gegen radioaktiv markierte polyA⁺RNA aus verwundeten Kartoffelknollen.

Die Isolierung von Einzelstrang-DNA aus M13-Phagen (sie enthält den kodogenen Strang), die Fixierung dieser DNA auf Nitrocellulose und Hybridisierung gegen Kartoffelknollen-RNA sowie das Waschen und Freisetzen der selektierten RNA ist bei Maniatis et al. (1982) beschrieben. Die selektierte RNA sowie aus Kartoffelknollen isolierte polyA⁺RNA wurden in einem Kaninchen-Retikulozytenlysat (Pelham und Jackson, Eur. J. Biochem. 67, 247 (1976)) in Anwesenheit von ³⁵S-Methionin translatiert. Die ein- bzw. zweidimensionale elektrophoretische Auftrennung auf einem Polyacrylamid-Gel erfolgte nach Mayer et al. (1987).

### Beispiel 3

### Erstellen und Untersuchen einer genomischen Bank

### Isolierung der genomischen DNA:

Als genomische Kartoffel-DNA diente nach der Methode von Bedbrook (1981) isolierte Blatt-DNA der haploiden Linie AM 80/5793.

Diese DNA wurde vollständig mit EcoRI verdaut.

### Isolierung der Phagen-DNA:

EMBL4-DNA wurde mit EcoRI in drei Fragmente zerschnitten, wobei über gelelektrophoretische Auftrennung mit anschließender Fragmentisolierung die beiden Vektorarme von dem Mittelfragment getrennt werden konnten. Zusätzlich wurden auch kommerziell erhältliche und gereinigte EMBL4-Arme verwandt (Amersham).

### Ligation und Verpackung:

Nach erfolgter Ligation der EMBL4-Arme mit der EcoRI verdauten genomischen DNA wurde die ligierte, hochmolekulare DNA in vitro in Phagenköpfe verpackt (Hohn und Murray, Proc. Nat. Acad. Sci. USA, 74, 3259-63, (1977); Hohn, Meth. Enzym. 68, 299-309 (1979)). Das Verpackungsmaterial stammt aus einem "Lambda in vitro packaging kit" der Firma Amersham. Die genomische Bank wurde mit einer Konzentration von 25000 plaques pro Platte (25x25cm) ausplattiert.

### Plaquehybridisierung:

Das Auffinden cDNA-homologer Lambda-Klone erfolgte durch Plaquehybridisierung nach Benton und Davies, Science 196, 180 (1977) mittels radioaktiv markierter cDNA. Positiv hybridisierende Plaques wurden vereinzelt und nochmalig getestet.

### DNA-Präparation rekombinanter Phagen:

20 ml einer C600-lysierten Bakterienkultur wurden mit Chloroform versetzt, um nach anschließender Zentrifugation einen bakterienfreien Überstand zu erhalten. Das Sedimentieren der Phagen aus dem Überstand erfolgte durch 4-stündiges Zentrifugieren bei 10000 upm. Das Phagen-Sediment wurde in 500 »l Phagen-Puffer (10 mM Tris-HCl, pH 8,0, 10 mM MgCl₂) aufgenommen und mit DNase und RNase behandelt. Nach der Extraktion der DNA durch mehrmaliges Phenolisieren, wurde die Phagen-DNA EtOH-präzipitiert, mit 70 %-igem EtOH gewaschen und in TE aufgenommen.

### Beispiel 4

### Erzeugung von Deletionsmutanten nach Exonuklease-III-Behandlung

Das zu untersuchende Fragment wurde nach Henikoff et al., Gene 28, 351-359, (1984) in beiden Orientierungen in M13mp19 kloniert. Da die Exonuclease III 5′-überstehende Enden abverdaut, jedoch 3′-überstehende Enden verschont, wurden 20 »g der zu analysierenden DNA mit zwei Restriktionsenzymen verdaut, die auf den Seiten des klonierten Fragments je ein 5′- und ein 3′-Ende erzeugten. Da das 5′-Ende dem Fragment zugewandt war, konnte eine Exonuclease-Verdauung in das Fragment hinein erfolgen. Durch sukzessives Stoppen dieser Reaktion konnten jeweils 200 bp kleinere Fragmente isoliert werden, deren überstehende Enden durch eine nachfolgende S1-Behandlung in ligierbare glatte Enden umgewandelt wurden. Zuletzt folgte die Transformation dieser DNA in BMH 7118-Zellen mit anschließender Einzelstrang-DNA-Isolierung.

### Sequenzierung

Die Sequenzierung von Einzelstrang-DNA wurde nach der Kettenabbruch-Methode von Sanger et al., Proc. Natl. Acad. Sci. USA 74, 5463, (1977) durchgeführt. Die Auftrennung der Reaktionsansätze erfolgte auf 6 und 8 %-igen Sequenzgelen (Ansorge und de Mayer, J. Chromatogr. 202, 45-53 (1980); Ansorge und Barker, J. Biochem. Biophys. Meth. 9, 33 (1984)).

### Klonierung und Sequenzierung von wun1-25A2

Das etwa 711 bp große Insert des Klons wun1-25A2 wurde über die PstI-Schnittstellen in den M13mp19-Vektor kloniert, wobei die Orientierung des Inserts über die asymmetrische XhoI-Schnittstelle ermittelt wurde. Die in der Orientierung des Inserts unterschiedlichen Klone 25A2-4-mp19 und 25A-5-mp19 wurden mit KpnI/XbaI geschnitten, um eine bidirektionale ExonukleaseIII-Verdauung in das Fragment hinein zu ermöglichen. Verschieden große Deletionsklone konnten durch sukzessives Stoppen der Exonukleasereaktion erhalten werden, die als gereinigte Einzelstrang-DNA zur Sequenzierung nach der Methode von Sanger et al. (1977) dienten.

### Beispiel 5

### S1-Analyse

Das Prinzip der S1-Nuklease-Kartierung zur Bestimmung des Transkriptionsstartpunkts basiert darauf, daß durch eine Hybridisierung von Einzelstrang-DNA aus dem 5′-Bereich des wun1-Gens mit wun1-mRNA nur die Bereiche vor einem Abbau der einzelstrangspezifischen Nuklease S1 geschützt sind, die aufgrund ihrer Homologie einen Doppelstrang ausbilden können. Die Größe des geschützten DNA-Fragments kann auf einem Sequenzgel ermittelt werden und somit der Transkriptionsstart zurückverfolgt werden.

Die Bestimmung des Transkriptionsstartpunktes wurde nach Berk und Sharp, Proc. Natl. Acad. Sci. USA 75, 1274 (1987), durchgeführt. Dazu wurde aus dem Plasmid pLS000 mit EcoRI und XhoI das 1,2 kb Fragment isoliert und durch Behandlung mit Phosphatase dephosphoryliert. Anschließend erfolgte eine radioaktive Markierung der 5′OH-Enden durch die Kombination von Polynukleotidkinase und y-³²-P-ATP. Nach Denaturierung der DNA-Fragmente wurden diese mit 50 »g Gesamt-RNA hybridisiert (Hybridisierungspuffer: 80 % Formamid, 0,4 mM NaCl, 40 mM PIPES pH 6,4 und 1 mM EDTA). Diese Bedingung begünstigt RNA-DNA-Hybride im Vergleich zu DNA-DNA-Hybriden (Casey und Davidson, Nucl. Acids Res. 4, 1539-1552 (1977)). Die Hybridisierungstemperatur beginnt bei 80°C und wird über Nacht auf 40°C gesenkt. Eine anschließende S1-Nuclease Verdauung (120 U/ml) entfernt ungepaarte Stränge. Es wurde erwartet, daß die im 5′-untranslatierten Bereich des wun1-Gens gelegene und radioaktiv markierte XhoI Schnittstelle durch ihre Homologie zur mRNA geschützt ist. Zuletzt wird der S1-geschützte DNA-Strang auf einem Sequenzgel elektrophoretisch aufgetrennt, wobei als Längenvergleich die Sequenz eines bekannten DNA-Fragments dient.

### Beispiel 6

### Transiente Expression in Kartoffelprotoplasten

Je 20 »g hochgereinigte (CsCl-Gradient) Plasmid-DNA wird in PEG/CaCl₂-behandelte Kartoffelprotoplasten transformiert (Lipphardt, Dissertation, Köln, 1988). Die Protoplasten stammen aus einer Kartoffelstengel-Suspensionskultur der Sorte Datura.

### Transiente Expression in Reisprotoplasten

20 »g hochgereinigte (CsCl-Gradient) Plasmid-DNA werden nach einer modifizierten Methode von Lörz et al., Mol. Gen. Genet. 199, 178-182 (1985), transformiert. Verändert wurden die Konzentrationen an Polyethylenglycol und Osmotikum.

### Beispiel 7

### DNA-Transfer in Agrobakterien

### Konjugation:

Die in E. coli klonierte DNA wurde nach der von Van Haute et al. (1983) beschriebenen Methode mittels des Helferstammes GJ23 in den Agrobakterienstamm 3850ₖₘ transferiert.

### DNA-Analyse von Agrobakterien:

Die Überprüfung des DNA-Transfers in das Agrobacterium erfolgte durch die Isolierung von Agrobakterien DNA nach der von Murray und Thompson, Nucl. Acid Res. 8, 4321-4325 (1980) geschilderten Methode. Restriktionsspaltung der DNA, der Transfer auf Nitrocellulose und die Hybridisierung gegen die entsprechende radioaktive Sonde gaben Aufschluß über einen erfolgreichen DNA-Transfer ins Agrobacterium.

### Beispiel 8

### Transformation von Tabak

### Anzucht der Agrobakterien:

Die zur Infektion benötigten Agrobakterien 3850_{Km} wurden in selektivem Antibiotika-Medium angezogen (Zambrisky et al., 1983) durch Zentrifugation sedimentiert und in YEB-Medium ohne Antibiotika gewaschen. Nach erneuter Sedimentation und Aufnahme in YEB-Medium konnten die Bakterien zur Infektion verwandt werden.

### Blattscheiben-Infektion:

Für die Blattscheiben-Infektion wurden sterile Blätter der Tabaklinien SNN und W38 verwendet. Etwa 1 cm große Blattstücke wurden durch 10-minütige Inkubation in 0,1 % HgCl₂, 0,1 % SDS sterilisiert und dreimal in sterilem Wasser gewaschen. Es folgte das Eintauchen in eine wie oben beschriebene Agrobakteriensuspension mit anschließender Überführung auf 3MS-Medium. Nach 2-tägiger Inkubation bei 16 h unter Licht und bei 25 bis 27°C wurden die Blattstücke mehrmals in flüssigem 3MS-Medium gewaschen und auf 3MSC-Medium überführt. Nach 4-6 Wochen erscheinende Sprosse wurden abgeschnitten und auf 2MSC-Medium inkubiert. Als weiterer Nachweis einer erfolgreichen Transformation wurde der Nopalin-Test durchgeführt.

### Analyse der transformierten Pflanzen

### Nachweis von Nopalin-Synthase-Aktivität:

Der Nachweis von Nopalin-Synthase-Aktivität in transformierten Pflanzenblättern wurde nach Otten und Schilperoort, Biochem. Biophys. Acta 527, 497 (1978) durchgeführt.

### Nachweis von NPT-II-Aktivität:

Die NPT-II-Aktivität in transformierten Pflanzen wurde nach Reiss et al., Gene 30, 217-223 (1984) und Schreier et al., EMBO J. 4, 25-32 (1985) nachgewiesen.

### Nachweis von CAT-Aktivität:

Chloramphenicol-Acetyl-Transferase(CAT)Aktivität wurde nach den Methoden von Velten und Schell, Nuc. Acids, Res. 13, 6981-6997 (1985) und Herrera-Estrella et al., EMBO J. 2, 987 (1983) nachgewiesen.

### Beispiel 9

Optimierung des wun1-Promotors durch Fusion mit dem TR-Promotor (TR1′wun1).

Bezüglich der verwendeten Medien wird auf die Beispiele 1-8 verwiesen.

### 2.1.4. Stämme und Vektoren

Agrobakterienstämme GV3101pmp90RK (Koncz, C., und Schell, J. "The promotor of TL-DNA gene 5 controls the tissue-specific expression of chimaeric genes carried by a novel type of Agrobacterium binary vetor", (1986), Mol. Gen. Gent. 204:383-396).
- Plasmide:: pPCV720 (bisher nicht publiziertes Plasmid von Dr. Koncz, MPI f. Züchtungsforschung, Köln)
35S-GUS = pRT99-GUS (Töpfer, R., Schell, J. und Steinbiß, H.H. "Versatile cloning vectors for transient gene expression and direct gene transfer in plant cells." (1988)
wun1-GUS (Siebertz, B., Logemann, J., Willmitzer, L., Schell, J. "Cis-analysis of the woundinducible promoter wun1 in transgenic tobacco plants and histochemical localisation of its expression". The Plant Cell, im Druck (1989)
MAS1' (TR1')-wun1-GUS (Logemann et al., im Druck)
MAS1' (TR1')-GUS (Logemann et al., im Druck)
- Pflanzen:: Nicotiana tabacum Wisconsin 38 (W38)

### Methoden

Alle molekularbiologischen Standard-Methoden, wie z. B. Restriktionsanalyse, Plasmidisolierung, Minipräparation von Plasmid-DNA, Transformation von Bakterien u.s.w. sind, sofern nicht anders angegeben, wie bei Maniatis et al., (1982) beschrieben durchgeführt worden.
Verwundetes und unverwundetes Pflanzengewebe wurde wie in den Beispielen 1-8 beschrieben hergestellt.

Bezüglich der RNA-Isolierung, der "Northern-Blot"Analyse, der DNA-Isolierung, der transienten Expression in Reisprotoplasten, der "Southern-Blot"Analyse und der DNA-Analyse von Agrobakterien wird ebenfalls auf die Beispiele 1-8 verwiesen.

### DNA-Transfer in Agrobakterien

### Transformation

Die in E. coli klonierte DNA wurde nach der von Van Houte, E., Joos, H., Maes, M., Warren G., Van Montagu, M., Schell, J. "Intergenic transfer and exchange recombination of restriction fragments cloned in pBR322: a novel strategy for reversed genetics of Ti-plasmids of Agrobacterium tumefaciens", EMBO J., 2:411-418 beschriebenen Methode mittels des Helferstammes S17-1 in den Agrobacterienstamm GV3101pmp90RK transferiert.

### Transformation von Tabak

### Anzucht der Agrobakterien

Die Agrobakterien GV3101pmp90RK, die das gewünschte Plasmid enthalten, wurden in selektivem Antibiotika-Medium (Hygromacin) angezogen, durch Zentrifugation sedimentiert und in YEB-Medium (Maniatis et al, 1982) ohne Antibiotika gewaschen. Nach erneuter Sedimentation und Aufnahme in YEB-Medium konnten die Bakterien zur Infektion verwandt werden.

### Blattscheiben-Infektion

Es wurde wie in Beispiel 8 beschriben gearbeitet, jedoch ohne Anwendung des Nopalin-Tests.

### Analyse der transformierten Pflanzen

### Nachweis von GUS-Aktivität

A: Der fluoromoetrische Nachweis von beta-glucoronidase-Aktivität (GUS-Aktivität) erfolgt nach de Mehode von Jefferson, A.R., Kavanagh, T.A., Bevan, M.W. "Gus-Fusions: ß-Glucuronidase as a sensitive and versatile gene fusion marker in higher plants", EMBO J. 6:3901-3907 (1987).
B: Der färbetechnische Nachweis von GUS-Aktivität in Geweben efolgte mittels einer X-Gluc Färbelösung. X-Gluc Lösung: 1-2 mM X-Gluc (5-Bromo-4-chloro-3-indolyl-glucuronid), und Dimethylformamid werden in 50 mM Phosphat-Puffer pH 7,0 gelöst.

### - Verwundetes Gewebe:

Dünne Gewebeschnitte werden inkubiert wie unter "Verwundetes Pflanzengewebe" beschrieben und anschließend in X-Gluc Lösung über Nacht bei 37°C gefärbt. Zuletzt wird durch Zugabe von 100% Methanol bei 60°C das Chlorophyll entfernt.

### -Unverwundetes Gewebe:

Dünne Gewebeschnitte werden in X-Gluc Lösung inkubiert, die zusätzlich den translationellen Inhibitor Cycloheximid (1,8 mM) enthält. Inkubationsdauer und Methanol-Behandlung ist identisch mit dem verwundetem Gewebe.

### -X-Gluc Färbung von Pollen:

Pollen wurden über Nacht bei 28°C in 30%iger Saccharose inkubiert, was zur Ausprägung von Pollenschläuchen führt. Anschließend wird doppelt konzentrierte 30%ige X-Gluc Lösung addiert und der Pollen über Nacht bei 28°C inkubiert. Alternativ werden Pollenkörner direkt in X-Gluc inkubiert, ohne daß es zur Ausprägung von Pollenschläuchen kommt.

### Ergebnisse

### Organisation von MAS1'-wun1-Gus, MAS1'-GUS und wun1-GUS (Fig. 12).

### wun1-GUS:

1022 bp des wun1-Promotors und 178 bp des wun1 5' untranslatierten Bereichs werden transkriptionell mit dem beta-Glucuronidase-Gen (GUS) fusioniert. Als Terminationssequenz dient das poly-Adenylierungssignal (pA) des Nopaline-synthase-Gens (NOS).

### MAS1'-GUS:

Der MAS1'-Promotor wird transkriptionell mit dem GUS-Gen fusioniert und durch das pA-Signal des NOS-Gens terminiert.

### MAS1'-wun1-GUS:

Hinter den 1'-Promotor des MAS-Promotors ist das pA-Signal des Octopine-synthase-Gens (OCS) kloniert, um eine Transkription durch den wun1-Promotor hindurch zu verhindern. Hinter dem OCS pA-Signal liegt der wun1-Promotor (1022 bp), der (in Homologie zu der wun1-GUS Konstruktion) über 179 bp des untranslatierten 5'Bereichs des wun1-Gens mit dem GUS-Gen transkriptionell fusioniert vorliegt. Als Terminationssequenz für das GUS-Gen dient das pA-Signal des 35-S Gens. Diese Konstruktion (MAS1'-wun1-GUS) liegt in dem binären Vektor pPCV720 vor, der sich selbst sowohl in E.coli als auch in Agrobakterien (mit Hilfe eines Helferplasmids) replizieren kann.

### RNA-Analyse von MAS1'-wun1-GUS transgenem Tabak

RNA aus verwundeten und aus unverwundeten Blättern verschiedener MAS1'-wun1-GUS transgener Tabakpflanzen sowie aus wun1-GUS und 35S-GUS Blättern wurde isoliert und gegen radioaktiv markierte GUS-DNA hybridisiert.
Wie der Northers-Blot in Fig. 13 zeigt, ist 1. die GUS-mRNA in transgenen MAS1'wun1-GUS-Blättern genauso groß wie in wun1-GUS transgenen Blättern. Offensichtlich wird in beiden Konstrukten der gleiche Transkriptionsstart benutzt.
2. die Menge an RNA ist sowohl bei MAS1'-wun1-GUS-Pflanzen als auch bei wun1-GUS Pflanzen in verwundeten Blättern höher als in unverwundeten Blättern. Die für wun1-GUS bereits bekannte Verwundungsinduzierbarkeit bestätigt sich somit auch für MAS1'-wun1-GUS.
3. bei einem mengenmäßigen RNA-Vergleich ist zu erkennen, daß in verwundeten MAS1'-wun1-GUS transgenen Blättern teilweise mehr RNA zu finden ist als in 35S-GUS transgenen Blättern. Da der 35S-Promotor zu einem der stärksten in Pflanzen aktiven Promotoren zählt, ist der MAS1'-wun1-Promotor ebenfalls so einzuschätzen.

### Analyse der GUS-Aktivität in transgenem Tabak

Eine quantitative Analyse der GUS-Aktivität transgener TR1'-wun1-GUS Pflanzen zeigt, daß der MAS2'1'-wun1-Promotor in transgenen Blättern durch Verwundung um den Faktor 2 bis 13fach induzierbar ist (im Durchschnitt etwa 6fach). Dies entspricht ungefähr der Wundinduzierbarkeit des wun1-Promotors in transgenen Blättern (Logemann J., Lipphardt S., Lörz H., Häuser I., Willmitzer L. und Schell J. "5'upstream sequences from the wun1-gene are responsible for gene activation by wounding in transgenic plants" The Plant Cell 1:151-158 (1989A)). Analog zu transgenen wun1-GUS Tabakpflanzen ist die Wundinduzierbarkeit in den oberen, jungen Blättern am höchsten, in unteren, alten Blättern am niedrigsten. Ursache für diese Unterschiede in der Induzierbarkeit ist die von oben nach unten zunehmende Aktivität des MAS1'1-wun1-Promotors in unverwundetem Gewebe. In alten Blättern von alten Pflanzen ist die MAS1'-wun1-Promotor Aktivität in unverwundetem Zustand bereits so hoch, daß sie durch eine Verwundung nicht mehr weiter induziert werden kann (analog zu wun1-GUS Pflanzen).

Ein relativer Vergleich von Promotor-Aktivitäten in transgenen Tabak-Blättern und -Stengeln zeigt, daß der wun1-Promotor etwa 10fach stärker als der MAS1'-Promotor ist. Der MAS1'-wun1GUS Promotor ist wiederum 10fach stärker als der wun1-Promotor. Somit ist der MAS1'-wun1-Promotor etwa 100fach stärker als der MAS1'-Promotor.

### Lokalisierung von GUS-Aktivität in transgenem Tabak

### -Blatt, Stengel:

Die GUS-Aktivität in den Gewebsschnitten von verwundeten und unverwundeten Blättern und Stengeln von wun1-GUS und MAS1'-wun1-GUS transgenem Tabak wurde durch die Verwendung des Substrates X-Gluc lokalisiert.

### wun1-GUS:

Wie anhand blaugefärbter Bereiche zu sehen ist, beschränkt sich die Aktivität des wun1-Promotors hauptsächlich auf die Epidermis (inclusive Trichome) und zu einem geringeren Anteil auf das Leitbündelsystem von verwundeten Blättern (W). In unverwundeten (NW) Blättern hingegen ist nur eine geringe wun1 Promotoraktivität in der Epidermis und keine Aktivität im Leitbündelsystem zu sehen. Die gleichen Ergebnisse werden auch mit Stengelquerschnitten erzielt.
Somit ist die wun1-Promotoraktivität als Epidermis-spezifisch in Blättern und Stengeln anzusehen.

In MAS1'wun1-GUS Pflanzen ist eine intensive Blaufärbung in dem Leitbündelsystem verwundeter Blattschnitte zu erkennen. In anderen Bereichen (Epidermis, Parenchym) ist die Färbung nur schwach. Unverwundete Blattschnitte zeigen eine geringe Blaufärbung im Leitbündelsystem und keine Färbung in der Epidermis. Die gleichen Ergebnisse werden auch mit Stengelquerschnitten erzielt.
Offensichtlich handelt es sich bei MAS1'-wun1-GUS um einen in Blättern und Stengeln Leitbündelsystem-spezifisch exprimierenden Promotor.

Als Kontrolle dienten Blattquerschnitte von MAS1'GUS Pflanzen. Aufgrund der schwachen Promotoraktivität des MAS1'-Promotors (10fach schwächer als der wun1-Promotor) ist weder in verwundeten noch in unverwundeten Blattquerschnitten eine Blaufärbung erkennbar.

### -Anthere:

Eine X-Gluc Färbung von Antherenquerschnitten sowie von Pollen zeigt folgende Charakteristika, die sowohl für wun1-GUS als auch für MAS1'-wun1-GUS zutreffen:
Eine Blaufärbung (und damit GUS-Aktivität) wird im Stomium (natürliche Perforationsstelle der Anthere, um den Pollen zu entlassen) und im Pollen nachgewiesen. In separierten ungekeimten und gekeimten Pollen ist eine Blaufärbung im Pollenkorn und im Pollenschlauch zu erkennen.

### Transiente Expression mit Reis-Protoplasten

Die Konstruktionen MAS1'-wun1-GUS, wun1-GUS, MAS1'-GUS und 35S-GUS wurden zu transienten Expressionstudien mit Reis-Protoplasten der Sorte (Oryza sativa japonica c.v. Taipai) benutzt. Wie Tab. 1 zeigt, unterscheidet sich die Aktivität von MAS1'-GUS nicht signifikant von der Kontrolle (GUS-Aktivität von Reis-Protoplasten, die ohne DNA transformiert wurden). wun1-GUS und 35S-GUS zeigen eine 2-3fach höhere Aktivität als die Kontrolle. MAS1'-wun1-GUS DNA hingegen zeigt im Durchschnitt eine 57fach höhere Aktivität als die Kontrolle und ist somit ein hochaktiver Promotor in Reisprotoplasten.

**Tabelle 1**

| Konstrukt | GUS-Aktivität nmol MU/mg)min | relative GUS-Aktivität |
|---|---|---|
| MAS-GUS | 31 | 1,2 |
| wun-GUS | 59 | 2,3 |
| MAS-wun-GUS | 1416 | 56,7 |
| 35S-GUS | 66 | 2,6 |
| Kontrolle | 25 | 1,0 |

### Legende zu Tab.1:

Die angegebene GUS-Aktivität wurde aus 5 unabhängigen Versuchen ermittelt.

Die Aussage der Tab.1 läßt sich durch das Anfärben der Protoplasten mit X-Gluc belegen. Nur mit MAS1'-wun1-GUS transformierte Protoplasten zeigen eine intensive Blaufärbung.

## Patentansprüche

1. DNA-Sequenz aus Solanum tuberosum, die durch Verletzung und/oder Infektion mit pathogenen Keimen stimuliert wird, umfassend die folgenden Nukleotide :

5' 1 AAATTTTATACATACATACAAACACAATCATTGATACATAACAA
45 TGATGTACATATATACAATTATCTAACCGATATATATATATATATATATATATATATATA
105 TCACCTCTCTCCACTCTCTGCCCTCTCTTCATTCTCTCCCAATCTCGCTCACCACTCTAG
165 CCTAACATAGAGAACATATACATATACAAACACAATTTTCATAGACACAAATGGAATTAA
225 TACCTGATTTATATAAATCGCCGTGATTTATACAAATCAGATGCTCCATAACAAACATAA
285 TTTGACCCATGGAGTGCATATAACAAAATTGTAGCTATAGAACGCTAATATGTTTTTCTT
345 AATCTTTGTTATTCCTAAAATTTACTCATAATAATACTCTTTATAAAAGCATAAGCTGGT
405 TTGGTTTAGGGTTAGAGTATTCTCTAAAAATTCTAATTGAAATCAAATACATCTTATAGA
465 ATCCAAATTAGAATTGAACACGTCTTGTAGAGTCCCATAAATTTTTAATGTCTACAATGT
525 AATATCGTTAAAATATTTTAATATCTTGTTGAAATATAATTTTTTATTTAGTAAAATAAT
585 ATGAGAATTAATTTTTTTTATTAATCTTCACAACTATGATTTTTTTAAAATTTCATGTAA
645 ATATATGGGCTAAGATTGTGAGCCAACTGGTCAAAACTCAAAAGTTAGTCGAGTTTGAAT
705 GAAGTTAAAATTAAAAGTATTGTTGTCATAACTCATATGTTGCAAGTTGCAACTGTGTGT
765 ATAAGGTCAAAAAAGGTATGCTTGAAAGTTGAAACTTTAGATATGACGATCATCTTCGTG
825 GGCCCTACCTAAAATAAAACGTCTCTTCATCATCCGAATATCACATCATCACGTAATCCC
885 CGAGCACGTGGAATGGCGCGTAAATATCATGTCGCCCTTTAAACCTAAATACACCTACTA
945 TTCACCTATAATTTCCAAACTACCCTTCCAACGTCCCTATATATTCCCCACATCACACCT
1005 CTTTCTTCATTACCTACCATACCTTCTTCTCTCATCCTTCATAGCTAATAATATCATCTT
1065 CTGTTTTTACTGAACTGGCTAACTCTCAGTTAACTCTGGAGGAAACAACAAACAAAGGGG
1125 TGGTGCTCGCCCTATATGAAGCCTTGAGCTCACACGACGTCGTTCAGGTCCAGAAACTCC
1185 TGGCCTCCGACCTCGAGTGGTGGTTCCATGGTCCTCCTTCTCATCAATTTTTGATGCAAA
M Q I
1245 TACTCACCGGCACTGCTAAATTCGATAACGCCTCTTTTCAATTCCTTCATAAGACCATTG
L T G T A K F D N A S F Q F L H K T I D
1305 ACGTATTCGGTTCCGTTGTTCTCGTCGAAGGCTGTGACCCGACCCGATCTATTACTTGGG
V F G S V V L V E G C D P T R S I T W V
1365 TTCACGCCTGGACTGTTACGGATGGGGTAATTACCCAGGTTAGGGAGTATTTCAATACCT
H A W T V T D G V I T Q V R E Y F N T S
1425 CACTTACTGTCACCCGTTTTGGGAAATCGGATATTTCCTCAATTACGACTCTGCATTGCC
L T V T R F G K S D I S S I T T L H C P
1485 CATCTGTTTGGGAGAGTAGCTTACCAAATCGGGTCGGAAAATCTGTTCCGGGTCTTGTAT
S V W E S S L P N R V G K S V P G L V L
1545 TGGCTCTATAAGAAACGACCCGATTTGTGGCTGGCGTTATATCTTGTGTCTAGTAGGATG
A L END
1605 TAAGATTAACGCGGCTGGTTTGGGATTCTGTTGCTGCTGTTTGGTTTGTTTTTATTTTTT
1665 AAGTTGGGATTTGTGTTCTGTTTATTATTTGTTGTTGTGATTGAGTTAAAGAAGGGGCCA
1725 ATGATAATTGGATATACCTTTCTACTCAATAGATTGTCTAATTATGTGTTGGTTTGCAAT
1785 AAAAAGCATATTGATTGGCTGTTTACAAAATGTGTATATTTTTCAATTTGGTATTGCTTC
1845 TTGTTTTCAACGAATGACGATGGATACCGTGGAAACAATTTCATTGAAAGGCAAATCTTA
1905 TTATTAATGATGGAATCAGAATTTTTATTATGAGGTTTAAATAATTAAACATATAAAATA
1965 GTTAAATGAGGTTCGAAATCTATATAATGCATAAAAAAATAATTTTAACTATATATAAAA
2025 ATGTATAATTTTTTTGTCTGAGAATAGGAGGAACAATATATTTAAGAAGGAATCTTTGCTT
2085 GCTTAGGATAGCTTCACATCATACTTTTCCCACTACATAGGTAATAGGAAGGCATATGCT
2145 CTATTCCTTCATAACTTACTCTTGGTATTTCTTATCCTAATCGCCAAAAAAAGAGAGTAT
2205 AATTTTTATTTATAACACACTTTTTTATTTCCTATACAAGAATAAGTTGATTTTTCCATT
2265 TAAGTATAAACATCGAAACTTTTAGAGCGACTCTATAAGAGACTGCCCAATTTCATTGGG
2325 ATTCTACAAGATATTTTCATTGATTCTTGGAGACTACTTAATCT 2368

2. Promotorteil der DNA-Sequenz nach Anspruch 1 oder seine transkriptionell aktiven Bereiche.

3. Promotorteil der DNA-Sequenz nach Anspruch 1 oder seine transkriptionell aktiven Bereiche, der bzw. die mit dem Mannopin-Synthase-Promotor fusioniert ist, bzw. sind.

4. Strukturgenteil der DNA-Sequenz nach Anspruch 1 oder seine transkriptionell aktiven Bereiche.

5. Verwendung der DNA-Sequenz nach Anspruch 1 oder der Promotorteile nach Ansprüchen 2 und 3 zur Expression von Genprodukten in höheren Pflanzen bei Verletzung und/oder pathogenem Befall.

6. Verwendung nach Anspruch 5,
dadurch **gekennzeichnet,**
daß der Promotorteil der DNA-Sequenz nach Anspruch 2 oder 3 oder seine transkriptionell aktiven Bereiche mit einem Strukturgen anderer Herkunft fusioniert ist/sind.

7. DNA-Übertragungsvektor mit insertierter DNA-Sequenz nach einem der Ansprüche 1, 2, 3 und/oder 4.

8. DNA-Übertragungsvektor nach Anspruch 7,
dadurch **gekennzeichnet,**
daß er den Promotorteil oder seine transkriptionell aktiven Bereiche in Verbindung mit einem Strukturgen anderer Herkunft enthält.

9. Pflanzen oder Pflanzenmaterial enthaltend eine DNA-Sequenz nach Anspruch 1, 2,3 oder 4.

## Claims

1. DNA sequence of Solanum tuberosum, which is stimulated by means of wounding and/or infection with pathogenic germs, comprising the following nucleotides:

5' 1 AAATTTTATACATACATACAAACACAATCATTGATACATAACAA
45 TGATGTACATATATACAATTATCTAACCGATATATATATATATATATATATATATATATA
105 TCACCTCTCTCCACTCTCTGCCCTCTCTTCATTCTCTCCCAATCTCGCTCACCACTCTAG
165 CCTAACATAGAGAACATATACATATACAAACACAATTTTCATAGACACAAATGGAATTAA
225 TACCTGATTTATATAAATCGCCGTGATTTATACAAATCAGATGCTCCATAACAAACATAA
285 TTTGACCCATGGAGTGCATATAACAAAATTGTAGCTATAGAACGCTAATATGTTTTTCTT
345 AATCTTTGTTATTCCTAAAATTTACTCATAATAATACTCTTTATAAAAGCATAAGCTGGT
405 TTGGTTTAGGGTTAGAGTATTCTCTAAAAATTCTAATTGAAATCAAATACATCTTATAGA
465 ATCCAAATTAGAATTGAACACGTCTTGTAGAGTCCCATAAATTTTTAATGTCTACAATGT
525 AATATCGTTAAAATATTTTAATATCTTGTTGAAATATAATTTTTTATTTAGTAAAATAAT
585 ATGAGAATTAATTTTTTTTATTAATCTTCACAACTATGATTTTTTTAAAATTTCATGTAA
645 ATATATGGGCTAAGATTGTGAGCCAACTGGTCAAAACTCAAAAGTTAGTCGAGTTTGAAT
705 GAAGTTAAAATTAAAAGTATTGTTGTCATAACTCATATGTTGCAAGTTGCAACTGTGTGT
765 ATAAGGTCAAAAAAGGTATGCTTGAAAGTTGAAACTTTAGATATGACGATCATCTTCGTG
825 GGCCCTACCTAAAATAAAACGTCTCTTCATCATCCGAATATCACATCATCACGTAATCCC
885 CGAGCACGTGGAATGGCGCGTAAATATCATGTCGCCCTTTAAACCTAAATACACCTACTA
945 TTCACCTATAATTTCCAAACTACCCTTCCAACGTCCCTATATATTCCCCACATCACACCT
1005 CTTTCTTCATTACCTACCATACCTTCTTCTCTCATCCTTCATAGCTAATAATATCATCTT
1065 CTGTTTTTACTGAACTGGCTAACTCTCAGTTAACTCTGGAGGAAACAACAAACAAAGGGG
1125 TGGTGCTCGCCCTATATGAAGCCTTGAGCTCACACGACGTCGTTCAGGTCCAGAAACTCC
1185 TGGCCTCCGACCTCGAGTGGTGGTTCCATGGTCCTCCTTCTCATCAATTTTTGATGCAAA
M Q I
1245 TACTCACCGGCACTGCTAAATTCGATAACGCCTCTTTTCAATTCCTTCATAAGACCATTG
L T G T A K F D N A S F Q F L H K T I D
1305 ACGTATTCGGTTCCGTTGTTCTCGTCGAAGGCTGTGACCCGACCCGATCTATTACTTGGG
V F G S V V L V E G C D P T R S I T W V
1365 TTCACGCCTGGACTGTTACGGATGGGGTAATTACCCAGGTTAGGGAGTATTTCAATACCT
H A W T V T D G V I T Q V R E Y F N T S
1425 CACTTACTGTCACCCGTTTTGGGAAATCGGATATTTCCTCAATTACGACTCTGCATTGCC
L T V T R F G K S D I S S I T T L H C P
1485 CATCTGTTTGGGAGAGTAGCTTACCAAATCGGGTCGGAAAATCTGTTCCGGGTCTTGTAT
S V W E S S L P N R V G K S V P G L V L
1545 TGGCTCTATAAGAAACGACCCGATTTGTGGCTGGCGTTATATCTTGTGTCTAGTAGGATG
A L END

1605 TAAGATTAACGCGGCTGGTTTGGGATTCTGTTGCTGCTGTTTGGTTTGTTTTTATTTTTT
1665 AAGTTGGGATTTGTGTTCTGTTTATTATTTGTTGTTGTGATTGAGTTAAAGAAGGGGCCA
1725 ATGATAATTGGATATACCTTTCTACTCAATAGATTGTCTAATTATGTGTTGGTTTGCAAT
1785 AAAAAGCATATTGATTGGCTGTTTACAAAATGTGTATATTTTTCAATTTGGTATTGCTTC
1845 TTGTTTTCAACGAATGACGATGGATACCGTGGAAACAATTTCATTGAAAGGCAAATCTTA
1905 TTATTAATGATGGAATCAGAATTTTTATTATGAGGTTTAAATAATTAAACATATAAAATA
1965 GTTAAATGAGGTTCGAAATCTATATAATGCATAAAAAAATAATTTTAACTATATATAAAA
2025 ATGTATAATTTTTTGTCTGAGAATAGGAGGAACAATATATTTAAGAAGGAATCTTTGCTT
2085 GCTTAGGATAGCTTCACATCATACTTTTCCCACTACATAGGTAATAGGAAGGCATATGCT
2145 CTATTCCTTCATAACTTACTCTTGGTATTTCTTATCCTAATCGCCAAAAAAAGAGAGTAT
2205 AATTTTTATTTATAACACACTTTTTTATTTCCTATACAAGAATAAGTTGATTTTTCCATT
2265 TAAGTATAAACATCGAAACTTTTAGAGCGACTCTATAAGAGACTGCCCAATTTCATTGGG
2325 ATTCTACAAGATATTTTCATTGATTCTTGGAGACTACTTAATCT 2368 3'

2. Promoter element of the DNA sequence according to claim 1 or its transcriptionally active regions.

3. Promoter element of the DNA sequence according to claim 1 or its transcriptionally active regions, which is or are fused with the mannopinsynthase promoter.

4. Structural gene element of the DNA sequence according to claim 1 or its transcriptionally active regions.

5. Use of the DNA sequence according to claim 1 or the promotor elements according to claims 2 and 3 in the expression of gene products in higher plants in the case of wounding and/or pathogenic attack.

6. Use according to claim 5, characterized in that the promoter element of the DNA sequence according to claim 2 or 3 or its transcriptionally active regions is/are fused with a structural gene of another origin.

7. DNA transmission vector with inserted DNA sequence according to one of claims 1, 2, 3 and/or 4.

8. DNA transmission vector according to claim 7, characterized in that it contains the promoter element or its transcriptionally active regions in conjunction with a structural gene of another origin.

9. Plants or plant material containing a DNA sequence according to claim 1, 2, 3 or 4.

## Revendications

1. Séquence d'ADN de Solanum tuberosum, qui est stimulée par lésion et/ou infection avec des germes pathogènes, comprenant les nucléotides suivants :

5' 1 AAATTTTATACATACATACAAACACAATCATTGATACATAACAA
45 TGATGTACATATATACAATTATCTAACCGATATATATATATATATATATATATATATATA
105 TCACCTCTCTCCACTCTCTGCCCTCTCTTCATTCTCTCCCAATCTCGCTCACCACTCTAG
165 CCTAACATAGAGAACATATACATATACAAACACAATTTTCATAGACACAAATGGAATTAA
225 TACCTGATTTATATAAATCGCCGTGATTTATACAAATCAGATGCTCCATAACAAACATAA
285 TTTGACCCATGGAGTGCATATAACAAAATTGTAGCTATAGAACGCTAATATGTTTTTCTT
345 AATCTTTGTTATTCCTAAAATTTACTCATAATAATACTCTTTATAAAAGCATAAGCTGGT
405 TTGGTTTAGGGTTAGAGTATTCTCTAAAAATTCTAATTGAAATCAAATACATCTTATAGA
465 ATCCAAATTAGAATTGAACACGTCTTGTAGAGTCCCATAAATTTTTAATGTCTACAATGT
525 AATATCGTTAAAATATTTTAATATCTTGTTGAAATATAATTTTTTATTTAGTAAAATAAT
585 ATGAGAATTAATTTTTTTTATTAATCTTCACAACTATGATTTTTTTAAAATTTCATGTAA
645 ATATATGGGCTAAGATTGTGAGCCAACTGGTCAAAACTCAAAAGTTAGTCGAGTTTGAAT
705 GAAGTTAAAATTAAAAGTATTGTTGTCATAACTCATATGTTGCAAGTTGCAACTGTGTGT
765 ATAAGGTCAAAAAAGGTATGCTTGAAAGTTGAAACTTTAGATATGACGATCATCTTCGTG
825 GGCCCTACCTAAAATAAAACGTCTCTTCATCATCCGAATATCACATCATCACGTAATCCC
885 CGAGCACGTGGAATGGCGCGTAAATATCATGTCGCCCTTTAAACCTAAATACACCTACTA
945 TTCACCTATAATTTCCAAACTACCCTTCCAACGTCCCTATATATTCCCCACATCACACCT
1005 CTTTCTTCATTACCTACCATACCTTCTTCTCTCATCCTTCATAGCTAATAATATCATCTT
1065 CTGTTTTTACTGAACTGGCTAACTCTCAGTTAACTCTGGAGGAAACAACAAACAAAGGGG
1125 TGGTGCTCGCCCTATATGAAGCCTTGAGCTCACACGACGTCGTTCAGGTCCAGAAACTCC
1185 TGGCCTCCGACCTCGAGTGGTGGTTCCATGGTCCTCCTTCTCATCAATTTTTGATGCAAA
M Q I
1245 TACTCACCGGCACTGCTAAATTCGATAACGCCTCTTTTCAATTCCTTCATAAGACCATTG
L T G T A K F D N A S F Q F L H K T I D
1305 ACGTATTCGGTTCCGTTGTTCTCGTCGAAGGCTGTGACCCGACCCGATCTATTACTTGGG
V F G S V V L V E G C D P T R S I T W V
1365 TTCACGCCTGGACTGTTACGGATGGGGTAATTACCCAGGTTAGGGAGTATTTCAATACCT
H A W T V T D G V I T Q V R E Y F N T S
1425 CACTTACTGTCACCCGTTTTGGGAAATCGGATATTTCCTCAATTACGACTCTGCATTGCC
L T V T R F G K S D I S S I T T L H C P
1485 CATCTGTTTGGGAGAGTAGCTTACCAAATCGGGTCGGAAAATCTGTTCCGGGTCTTGTAT
S V W E S S L P N R V G K S V P G L V L
1545 TGGCTCTATAAGAAACGACCCGATTTGTGGCTGGCGTTATATCTTGTGTCTAGTAGGATG
A L END

1605 TAAGATTAACGCGGCTGGTTTGGGATTCTGTTGCTGCTGTTTGGTTTGTTTTTATTTTTT

1665 AAGTTGGGATTTGTGTTCTGTTTATTATTTGTTGTTGTGATTGAGTTAAAGAAGGGGCCA

1725 ATGATAATTGGATATACCTTTCTACTCAATAGATTGTCTAATTATGTGTTGGTTTGCAAT

1785 AAAAAGCATATTGATTGGCTGTTTACAAAATGTGTATATTTTTCAATTTGGTATTGCTTC

1845 TTGTTTTCAACGAATGACGATGGATACCGTGGAAACAATTTCATTGAAAGGCAAATCTTA

1905 TTATTAATGATGGAATCAGAATTTTTATTATGAGGTTTAAATAATTAAACATATAAAATA

1965 GTTAAATGAGGTTCGAAATCTATATAATGCATAAAAAAATAATTTTAACTATATATAAAA

2025 ATGTATAATTTTTTTGTCTGAGAATAGGAGGAACAATATATTTAAGAAGGAATCTTTGCTT

2085 GCTTAGGATAGCTTCACATCATACTTTTCCCACTACATAGGTAATAGGAAGGCATATGCT

2145 CTATTCCTTCATAACTTACTCTTGGTATTTCTTATCCTAATCGCCAAAAAAAGAGAGTAT

2205 AATTTTTATTTATAACACACTTTTTTATTTCCTATACAAGAATAAGTTGATTTTTCCATT

2265 TAAGTATAAACATCGAAACTTTTAGAGCGACTCTATAAGAGACTGCCCAATTTCATTGGG

2325 ATTCTACAAGATATTTTCATTGATTCTTGGAGACTACTTAATCT 2368

3'

2. Promoteur de la séquence d'ADN selon la revendication 1 ou ses domaines actifs de transcription.

3. Promoteur de la séquence d'ADN selon la revendication 1 ou ses domaines actifs de transcription, qui est fusionné ou qui sont fusionnés avec le promoteur de la Mannopine-Synthase.

4. Gène de structure de la séquence d'ADN selon la revendication 1 ou ses domaines actifs de transcription.

5. Utilisation de la séquence d'ADN selon la revendication 1 ou du promoteur selon les revendications 2 et 3, pour l'expression de produits codés par des gènes dans les plantes supérieures, par lésion et/ou invasion par des agents pathogènes.

6. Utilisation selon la revendication 5, caractérisée en ce que le promoteur de la séquence d'ADN selon la revendication 2 ou 3 ou ses domaines actifs de transcription, est fusionné ou sont fusionnés avec un gène de structure d'une autre origine.

7. Vecteur de transfert d'ADN, dans lequel est inséré une séquence d'ADN selon l'une des revendications 1, 2, 3 et/ou 4.

8. Vecteur de transfert d'ADN selon la revendication 7, caractérisé en ce qu'il contient le promoteur ou ses domaines actifs de transcription,couplés avec un gène de structure d'une autre origine.

9. Plante ou matériel végétal contenant une séquence d'ADN selon les revendications 1, 2, 3 ou 4.
